# EUROPEAN PATENT APPLICATION

(11) **EP 3 550 010 A1**
(43) Date of publication of application: **09.10.2019**
(21) Application number: 17875942.9
(22) Date of filing: 20.10.2017
(51) Int. Cl.: C12M 1/34, G02B 21/00, G06T 7/00

(54) **INFORMATION PROCESSING DEVICE, OBSERVATION SYSTEM, INFORMATION PROCESSING METHOD, AND PROGRAM**

(30) Priority: 30.11.2016 JP 2016232132
(71) Applicant: Sony Corporation, Tokyo 108-0075 (JP)
(72) Inventor: SHINODA, Masataka, Tokyo 108-0075 (JP); ONUMA, Tomoya, Tokyo 108-0075 (JP)
(74) Representative: D Young & Co LLP
(86) International application number: PCT/JP2017/037939
(87) International publication number: WO 2018/100917

(57) **Abstract**

[Object] Provided are an information processing apparatus, an observation system, an information processing method, and a program, which are suitable for evaluating a fertilized ovum with a high accuracy.

[Solving Means] An information processing apparatus includes an image obtaining unit, an evaluation unit, and an image capturing controller unit. The image obtaining unit obtains an image of a cell, the image being captured by an image capturing unit. The evaluation unit evaluates a growth stage of the cell. The image capturing controller unit allows the image capturing unit to capture an image of the rotated cell, according to an evaluation result of the evaluation unit.

## Description

### Technical Field

The present technology relates to an information processing apparatus, an observation system, an information processing method, and a program, which are used for observing a culture cell.

### Background Art

Recently, studies have been conducted in the field of cell culture such as a fertility treatment or the breeding of livestock.

For example, in a case where the growth of a fertilized ovum according to external fertilization is observed, the fertilized ovum is accommodated in a culture vessel including an accommodation unit retaining the fertilized ovum, and the growth is observed. When a chronological change in the growth of the fertilized ovum is observed, an image of the fertilized ovum is captured by a camera, and thus, the image is obtained (for example, refer to Patent Literature 1). In the observation of the fertilized ovum, it is required to evaluate the fertilized ovum with a high accuracy.

### Citation List

### Patent Literature

Patent Literature 1: JP-A-2011-192109

### Disclosure of Invention

### Technical Problem

When the fertilized ovum is evaluated by using the captured image of the fertilized ovum, for example, the shape of the fertilized ovum after a blastocyst structurally has high asymmetry properties, and the appearance of the fertilized ovum is greatly different according to an observation angle. For this reason, it is not possible to perform sufficient evaluation only in an image that is captured from one observation angle.

An object of the present technology is to provide an information processing apparatus, an observation system, an information processing method, and a program, which are suitable for evaluating a fertilized ovum with a high accuracy.

### Solution to Problem

An information processing apparatus according to one embodiment of the present technology includes an image obtaining unit, an evaluation unit, and an image capturing controller unit.

The image obtaining unit obtains an image of a cell, the image being captured by an image capturing unit.

The evaluation unit evaluates a growth stage of the cell.

The image capturing controller unit allows the image capturing unit to capture an image of the rotated cell, according to an evaluation result of the evaluation unit.

According to this embodiment, when the cell is in a certain growth stage, the image of the rotated cell is captured, and thus, it is possible to obtain the image of the cell captured from a plurality of angles. Accordingly, in the evaluation of the fertilized ovum, it is possible to comprehensively evaluate the image of the cell captured from various angles, and to perform the evaluation with a high accuracy.

The information processing apparatus may further include a rotation controller unit.

The rotation controller unit controls a rotation mechanism that rotates the cell, according to the evaluation result of the evaluation unit.

Thus, the image of the cell rotated by the rotation mechanism may be captured.

The evaluation unit may evaluate the growth stage of the cell on a basis of the image obtained by the image obtaining unit.

Thus, the growth stage of the cell may be evaluated on the basis of the image of the cell.

The evaluation unit may evaluate the growth stage of the cell on a basis of a culture time of the cell.

The growth stage of the cell, for example, a fertilized ovum, follows approximately the same growth curve, insofar as the fertilized ovum is a normal fertilized ovum, and thus, it is possible to perform assumption according to an elapsed time (a culture time) from a fertilization date of the fertilized ovum. Therefore, it is possible to evaluate the growth stage of the cell, on the basis of the culture time of the cell.

When the evaluation unit evaluate that the cell is in a growth stage in which the cell has an asymmetric shape, the image capturing controller unit may allow the image capturing unit to capture the image of the rotated image.

Thus, in the growth stage in which the cell has the asymmetric shape, the image of the cell captured from the plurality of angles, is obtained, and thus, it is possible to more accurately grasp the growth of the cell in which various shapes are observed, according to the angle. Here, the asymmetric shape indicates a shape having high structural asymmetry properties, and indicates a shape when the appearance of the cell is greatly different according to an angle at which an approximately spherical cell is observed.

The growth stage in which the cell has the asymmetric shape may be a growth stage of a blastocyst.

After fertilization, the number of cells, for example, the number of fertilized ova of the mammals, increases according to segmentation such as a 2-cell period, a 4-cell period, an 8-cell period, and a 16-cell period, and eventually, the cells adhere to each other, and thus, become a morula. In a case where the growth further continues, a gap is generated in a cell cytoplasm, and thus, a blastocoele is formed, and becomes an early blastocyst, and in a case where the blastocoele expands, the blastocoele grows to a complete blastocyst. After the complete blastocyst, it is possible to identify an inner cell mass (hereinafter, referred to as ICM) that will be a fetus in the future, and a trophectoderm (hereinafter, referred to as TE). In the cell in the growth stage of the blastocyst, the appearance of the cell is greatly different according to an angle at which an approximately spherical cell is observed, and in a growth stage after the blastocyst in the shape having high structural asymmetry properties, the image of the rotated cell is captured, and the images of the cell from the plurality of angles are obtained, and thus, it is possible to accurately grasp the state of the growth of the cell.

The growth stage of the blastocyst may be a growth stage after a complete blastocyst.

In the fertilized ovum in the growth stage after the complete blastocyst, the ratio of the blastocoele in the fertilized ovum increases, and thus, the fertilized ovum is in a shape having structural asymmetry properties higher than those of the early blastocyst. Accordingly, for example, the image is easily sorted according to the position of the ICM in the fertilized ovum of the image, an image pattern is sorted into several patterns, on the basis of the position of the ICM in the fertilized ovum of the image, and thus, it is possible to observe the fertilized ova in different growth stages from approximately the same observation angle, and to accurately grasp a chronological change in the growth of the cell.

The cell may be a cell in the growth stage of the blastocyst having an inner cell mass, and the evaluation unit may sort the captured image of the rotated cell, according to an image capturing direction based on a position of the inner cell mass in the cell.

Thus, the image pattern is sorted into several patterns, on the basis of the position of the inner cell mass (ICM) in the fertilized ovum of the image, and thus, it is possible to observe the fertilized ova in different growth stages at approximately the same observation angle, and to accurately grasp a chronological change in the growth of the cell. For example, the image capturing direction of the cell may be sorted into three patterns of a front pattern, a lateral pattern, and an oblique pattern, on the basis of the time when the ICM is in a front position, as an example of sorting according to the image capturing direction, and the image capturing direction of the cell may be sorted by more specifically dividing the three image capturing directions according to the observation (an image capturing) angle, as another example.

The information processing apparatus may further include a storage unit that stores the image of the cell, the image being obtained by the image obtaining unit. The storage unit may store in advance a first feature amount of the cell in each growth stage, and the evaluation unit may extract a second feature amount of the image of the cell, the image being obtained by the image obtaining unit, and evaluate the growth stage of the cell on a basis of the first feature amount and the second feature amount.

The feature amount, for example, is a feature amount extracted on the basis of various growth stages of the cell, such as the size or the shape, and the sphericity of the fertilized ovum, the number of segmentations (a segmentation rate), the form of each blastomere and a balance thereof, fragmentation, the size or the shape of the ICM, the number of ICMs, and the density of the ICM. Thus, it is possible to evaluate the growth stage by referring to the first feature amount of each growth stage of the cell, stored in advance, and the second feature amount extracted from the obtained image.

The storage unit may store the arbitrarily selected cell as a supervised index.

For example, an expert (a user) such as an embryo culturer, may select a cell that is desired to be the supervised index, or in a case where the cell arbitrarily selected by the user is a cell of which the growth is particularly desired, in the evaluation of other cells by using the cell as the supervised index, it is possible to perform the evaluation with a higher accuracy.

The image capturing controller unit may control the image capturing unit such that the image capturing unit captures images of the plurality of cells, and the image capturing controller unit may control the image capturing unit such that the number of times of image capturing of the cell that is set as the supervised index is more than the number of times of image capturing of the cell that is not set as the supervised index.

Thus, a larger number of images of the cell that is set as the supervised index are obtained, and thus, it is possible to accumulate a large number of data items of the cell that is set as the supervised index, and an evaluation accuracy is improved by using the large number of data items.

The image capturing controller unit may control the image capturing unit such that the image capturing unit captures images of the plurality of cells, and the evaluation unit may set images of a part of cells in the plurality of cells, as a supervised image, and images of other cells in the plurality of cells, as a test image, and verify the supervised image by using the test image.

Accordingly, it is possible to evaluate the usefulness of the supervised image. Then, it is possible to set a cell that is evaluated as being useful, as the supervised index, to accumulate a larger number of data items of the cell that is the supervised index, and to perform the evaluation with a higher accuracy.

An observation system according to one embodiment of the present technology includes a culture vessel, an image capturing unit, an image obtaining unit, an evaluation unit, a rotation mechanism, and an image capturing controller unit.

The culture vessel includes a plurality of accommodation units in which a cell is accommodated.

The image capturing unit captures an image of the cell.

The image obtaining unit obtains the image of the cell, the image being captured by the image capturing unit.

The evaluation unit evaluates a growth stage of the cell.

The rotation mechanism rotates the cell in the accommodation unit, according to an evaluation result of the growth stage of the cell of the evaluation unit.

The image capturing controller unit controls the image capturing unit such that the image capturing unit captures an image of the cell rotated by the rotation mechanism.

According to this embodiment, for example, when it is evaluated that the cell is in a certain growth stage, the image is captured while rotating the cell by the rotation mechanism, and thus, it is possible to obtain the image of the cell captured from a plurality of angles. Accordingly, in the evaluation of the fertilized ovum, it is possible to comprehensively evaluate the image of the cell captured from various angles, and to perform the evaluation with a high accuracy.

The rotation mechanism may be a vibration apparatus that vibrates the culture vessel.

Thus, the cell may be rotated by vibrating the culture vessel.

The accommodation unit is capable of accommodating the cell and a liquid, the culture vessel may include the rotation mechanism, and the rotation mechanism may rotate the cell by generating a flow in the liquid in the accommodation unit.

An information processing method according to one embodiment of the present technology includes: evaluating a growth stage of a cell; and capturing an image of the rotated cell when it is evaluated that the cell is in a growth stage in which the cell has an asymmetric shape.

A program according to one embodiment of the present technology causes an information processing apparatus to execute an evaluation step and an image capturing step.

The evaluation step is a step of evaluating a growth stage of a cell.

The image capturing step is a step of capturing an image of a rotated cell when it is evaluated that the cell is in a growth stage in which the cell has an asymmetric shape.

### Advantageous Effects of Invention

As described above, according to the present technology, it is possible to specifically observe a change according to the growth of the cell, and to perform the evaluation with a high accuracy.

Note that, the effects described here are not necessarily limited, and may be any effect described in the present disclosure.

### Brief Description of Drawings

Fig. 1 is a plan view of a culture vessel according to each embodiment.
Fig. 2 is a perspective view of a dish holder retaining the culture vessel of Fig. 1.
Fig. 3 is a diagram describing a shape change according to the growth of a fertilized ovum.
Fig. 4 is a schematic view illustrating the configuration of an observation system according to a first embodiment, a fourth embodiment, a fifth embodiment, a seventh embodiment, and an eighth embodiment.
Fig. 5 is a block diagram illustrating the observation system according to the first embodiment, the fourth embodiment, the seventh embodiment, and the eighth embodiment.
Fig. 6 is a schematic view of an image of a fertilized ovum 16 in a growth stage after a complete blastocyst.
Fig. 7 is a diagram describing an image obtained by the observation system according to each of the embodiments.
Fig. 8 is a (first) flowchart of an image obtaining method of the observation system according to the first embodiment, a second embodiment, a third embodiment, and the eighth embodiment.
Fig. 9 is a (second) flowchart of the image obtaining method of the observation system according to the first embodiment, the second embodiment, the third embodiment, and the eighth embodiment.
Fig. 10 is a schematic view illustrating the configuration of the observation system according to the second embodiment.
Fig. 11 is a block diagram illustrating the observation system according to the second embodiment.
Fig. 12 is a schematic view illustrating the configuration of the observation system according to the third embodiment.
Fig. 13 is a block diagram illustrating the observation system according to the third embodiment.
Fig. 14 is a partially enlarged view of a culture vessel according to the third embodiment.
Fig. 15 is a diagram illustrating a configuration in the vicinity of a rotation unit of the observation system according to the third embodiment.
Fig. 16 is a (first) flowchart of an image obtaining method of the observation system according to the fourth embodiment.
Fig. 17 is a (second) flowchart of the image obtaining method of the observation system according to the fourth embodiment.
Fig. 18 is a block diagram illustrating the observation system according to the fifth embodiment.
Fig. 19 is a (first) flowchart of an image obtaining method of the observation system according to the fifth embodiment.
Fig. 20 is a (second) flowchart of the image obtaining method of the observation system according to the fifth embodiment.
Fig. 21 is a schematic view illustrating the configuration of an observation system according to a sixth embodiment.
Fig. 22 is a diagram describing an image obtaining method according to the seventh embodiment.
Fig. 23 is a diagram for describing an observation angle sorting code.
Fig. 24 is a diagram describing a correspondence between the observation angle sorting code and the captured image of the fertilized ovum.

### Mode(s) for Carrying Out the Invention

Hereinafter, embodiments according to the present technology will be described with reference to the drawings.

### (First Embodiment)

### <Configuration of Culture Vessel>

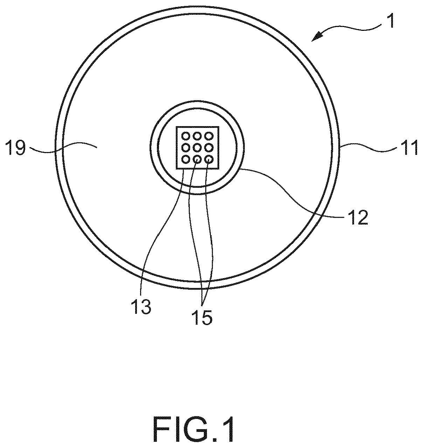

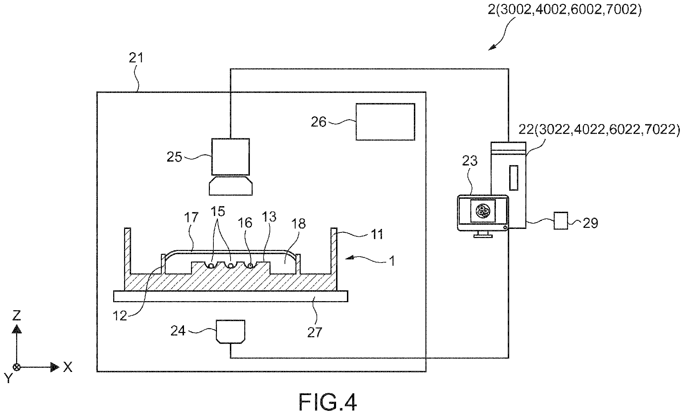

Fig. 1 is a plan view of a culture vessel (a dish). Fig. 4 is a schematic view describing a state in which the culture vessel is accommodated in an observation apparatus.

A culture vessel 1 is configured to be capable of accommodating a broth 18 and a cell 16, and has light transmissivity to the extent that an image of the cell 16 can be captured from the outside. Note that, the number of culture vessels 1 and the number of cells 16 of which the images can be simultaneously captured, are not limited.

In this embodiment, a living object in a stockbreeding field or the like, for example, a fertilized ovum of cattle will be described as an example of the cell 16 to be cultured (hereinafter, described as a fertilized ovum 16 by using the same reference numeral). It is not limited thereto, but for example, a biological sample or the like taken out from a biological body such as a stem cell, an immune cell, and a cancer cell, in the field of regeneration medicine or the like, can be exemplified as the cell to be cultured, and the present technology is effective with respect to a cell going through a growth stage in which the cell has a shape having high structural asymmetry properties, in which a three-dimensional image is required.

In addition, herein, the "cell" conceptually includes at least a single cell, and an aggregate of a plurality of cells.

As illustrated in Fig. 1, the culture vessel 1 includes a bottom portion 19, an outer-wall 11, an inner-wall 12, an accommodation unit 15, and a convex portion 13 for cell arrangement.

The culture vessel 1, for example, can be formed by using an inorganic material such as metal, glass, and silicon, and an organic material such as a polystyrene resin, a polyethylene resin, a polypropylene resin, an ABS resin, nylon, an acrylic resin, a fluorine resin, a polycarbonate resin, a polyurethane resin, a methyl pentene resin, a phenolic resin, a melamine resin, an epoxy resin, and a vinyl chloride resin. In this embodiment, a transparent culture vessel 1 formed of a polystyrene resin, is used. In this embodiment, a case where nine accommodation units 15 are arranged in one culture vessel 1, is illustrated as an example, but the number of accommodation units 15 is not limited thereto.

A plurality of accommodation units 15 are provided, and each of the accommodation units 15 is capable of holding one cell, here, the fertilized ovum 16 in a constant position while accommodating the fertilized ovum 16. In addition, a liquid is accommodated in each of the accommodation units 15, in addition to the fertilized ovum 16. The "liquid" is typically a broth suitable for culturing a cell, and hereinafter, will be described as the broth. As illustrated in Fig. 4, a broth 18 for culturing the fertilized ovum 16 is injected into a region surrounded by the inner-wall 12 in the accommodation unit 15. Further, in order to prevent the broth 18 from being evaporated, oil 17 is injected into the region surrounded by the inner-wall 12 to cover the broth 18.

A planar shape of the bottom portion 19 is a circular shape. The outer-wall 11 and the inner-wall 12 are concentrically formed, and the height of the inner-wall 12 is lower than the height of the outer-wall 11. The convex portion 13 for cell arrangement is arranged with a gap from the inner-wall 12 in the region surrounded by the inner-wall 12, in the center portion of the bottom portion 19. A planar shape of the convex portion 13 for cell arrangement is a rectangular shape.

### <Configuration of Dish Holder>

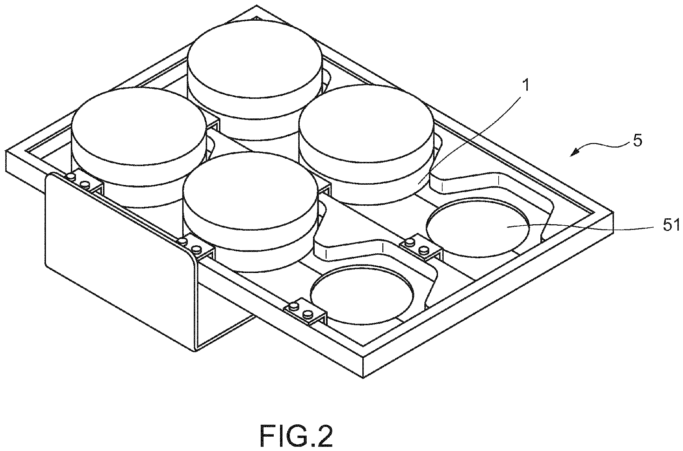

The culture vessel 1 described above, for example, can be retained by a dish holder, and can be retained in an observation apparatus described below. Fig. 2 is a perspective view of the dish holder.

As illustrated in Fig. 2, a dish holder 5, for example, is configured to be capable of retaining six culture vessels 1. A planar shape of the dish holder 5 is a rectangular shape, and the dish holder 5 includes six accommodation units 51 having a concave shape, which retain the culture vessel 1 and fix the position. A movement between an incubator and the observation apparatus, can be performed by the culture vessel 1 alone, or in a state where the culture vessel 1 is retained in the dish holder 5. In Fig. 2, a transparent lid formed of the same material is placed on the culture vessel 1.

### <Shape Change according to Growth of Fertilized Ovum>

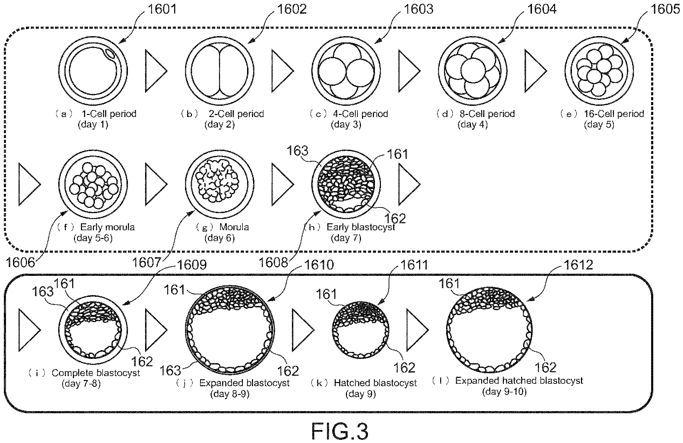

A shape change according to the growth of the fertilized ovum will be described by using Fig. 3.

Fig. 3 illustrates a general growth stage of the fertilized ovum 16 from the first day to the tenth day after fertilization. Fig. 3(a) is a fertilized ovum 1601 in a 1-cell period on the first day when the fertilization is confirmed. As illustrated in Fig. 3(b), the fertilized ovum 1601 is divided into two cells, and thus, becomes a fertilized ovum 1602 in a 2-cell period, on the second day from the fertilization. After that, in a case where the fertilized ovum 1602 smoothly grows, as illustrated in Fig. 3(c), Fig. 3(d), and Fig. 3(e), the number of fertilized ova 16 sequentially increases such as a fertilized ovum 1603 in a 4-cell period on the third day from the fertilization, a fertilized ovum 1604 in an 8-cell period on the fourth day from the fertilization, and a fertilized ovum 1605 in a 16-cell period on the fifth day from the fertilization.

After that, the cells adhere to each other, and become an early morula 1606 on the fifth day and the sixth day from the fertilization, as illustrated in Fig. 3(f), and become a morula 1607 on the sixth day from the fertilization, as illustrated in Fig. 3(g). In a case where the growth further continues, a gap is generated in a cell cytoplasm, and thus, a blastocoele is formed, and becomes an early blastocyst 1608 on the seventh day from the fertilization, as illustrated in Fig. 3(h). In a case where the blastocoele expands, a complete blastocyst 1609 is formed on the seventh day and the eighth day from the fertilization, as illustrated in Fig. 3(i). In a growth stage of the blastocyst (after 1608), it is possible to identify an inner cell mass 161 (hereinafter, referred to as ICM) that will be a fetus in the future, and a trophectoderm 162 (hereinafter, referred to as TE). In a growth stage of the early blastocyst 1608 and the complete blastocyst 1609, a transparent body 163 forming an outer shape of the fertilized ovum, is recognized. Further, the transparent body 163 is thinned, and thus, the fertilized ovum becomes an expanded blastocyst 1610 on the eighth day and the ninth day from the fertilization, and a hatched blastocyst 1611 in which the blastocyst is hatched from the transparent body, is formed on the ninth day from the fertilization, and an expanded hatched blastocyst 1612 is formed on the ninth day and the tenth day from the fertilization.

In the fertilized ovum 16, structural symmetry properties are comparatively high from the fertilization to a growth stage of the morula 1607, and the appearance is not greatly changed according to an observation angle. For example, in the fertilized ovum 16 in a growth stage of the 2-cell period and the 4-cell period, the appearance is different according to the observation angle, but the number of blastomeres is small in such cell periods, and for example, even in a case where the observation is performed from one observation angle, a segmentation state is comparatively easily grasped, a variation in the appearance according to the observation angle is small, and the appearance is not greatly changed according to the observation angle. In addition, in the fertilized ovum 16 of the morula from the 8-cell period, the structural symmetry properties become higher, and the appearance is approximately the same according to the observation angle.

However, in a case where the blastocoele is formed, and the fertilized ovum 16 is in the growth stage (1608 to 1612) of the blastocyst, the structural asymmetry properties become higher, and the appearance is greatly different according to the observation angle of the fertilized ovum 16. As illustrated in Figs. 3(i), 3(j), 3(k), and 3(l), in the fertilized ovum 16 in a growth stage after the complete blastocyst 1609, the ratio of the blastocoele in the fertilized ovum 16 increases, and thus, the ICM 161 biasedly exists in the spherical fertilized ovum 16.

In the present technology, an observation system is configured such that in a growth stage after the blastocyst, in which the fertilized ovum 16 has a shape having high structural asymmetry properties, the image of the rotated cell is captured, and the images of the cell, captured from a plurality of angles, are obtained. Thus, the fertilized ovum 16 after the blastocyst, of which the appearance is different according to the observation angle, can be observed by a plurality of images of the fertilized ovum 16, captured from the plurality of angles, and the state of the growth of the cell can be accurately grasped. Hereinafter, the observation system will be described.

### (First Embodiment)

### <Observation System>

An observation system of a first embodiment of observing the fertilized ovum 16 accommodated in the culture vessel 1 described above, will be described. Note that, in this embodiment, the incubator for culturing the fertilized ovum of the cattle, and the observation apparatus for performing the observation of the fertilized ovum are separate apparatuses, but a camera used for observing the fertilized ovum 16 may be provided in the incubator such that the fertilized ovum can be observed in the incubator.

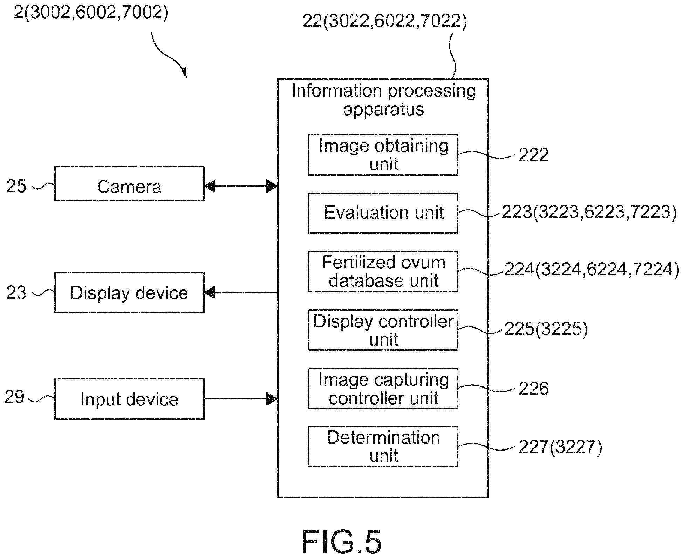

Fig. 4 is a schematic view illustrating the observation system. Fig. 5 is a block diagram illustrating the configuration of the observation system. In this embodiment and embodiments described below, it is described that one culture vessel is observed, as an example, but the observation system is configured to be capable of observing a plurality of fertilized ova at a time by providing one or a plurality of dish holders 5 in which a plurality of culture vessels 1 are placed, in the observation system.

As illustrated in Fig. 4, an observation system 2 includes an observation apparatus 21, an information processing apparatus 22, a display device 23, and an input device 29.

The observation apparatus 21 accommodates the culture vessel 1 in which the fertilized ovum 16 is accommodated, and observes the fertilized ovum 16. The culture vessel 1 is horizontally retained in the observation apparatus 21, and the fertilized ovum 16 is accommodated in each of the accommodation units 15 of the culture vessel 1. In the observation apparatus 21, a light source 24, a camera 25 as an image capturing unit, a temperature·humidity·gas controller unit 26, and a stage 27 are arranged.

When the image of the fertilized ovum 16 in the culture vessel 1 is captured by the camera 25, the light source 24 emits irradiation light to the culture vessel 1.

The camera 25 as the image capturing unit (hereinafter, described as the camera 25 by using the same reference numeral), for example, includes a visible light camera an image sensor such as a complementary metal-oxide semiconductor (CMOS) sensor or a charge coupled device (CCD) sensor. An infrared ray (IR) camera, a changing camera, or the like may be used instead of or in addition to the visible light camera.

The camera 25 captures the image of the fertilized ovum 16 in the culture vessel 1, and is arranged in the observation apparatus 21. The camera 25 includes a lens tube including a lens group movable in an optical axis direction (a Z-axis direction), a solid image capturing element such as a complementary metal oxide semiconductor (CMOS) or a charge coupled device (CCD), performing image capturing with respect to subject light penetrating through the lens tube, a driving circuit thereof, and the like. Note that, the camera 25 may be provided in the culture vessel 1 to be movable in the Z-axis direction and a horizontal direction (an XY-plane direction) in the drawing. In addition, the camera 25 may be configured to be capable of capturing not only a still image, but also a continuous image (a video).

In the camera 25, the number of times of image capturing, an image capturing timing, or the like is controlled by an image capturing controller unit 226 of the information processing apparatus 22 described below.

The temperature·humidity·gas controller unit 26 controls the temperature, the humidity, and the gas in the observation apparatus 21, and forms an environment suitable for culturing the fertilized ovum 16. The type of gas includes nitrogen, oxygen, carbon dioxide, and the like.

The input device 29 is connected to the information processing apparatus 22, and is a manipulation apparatus for inputting the scanning of a user. For example, a trackball, a touch pad, a mouse, a keyboard, or the like can be used as the input device 29.

The display device 23 outputs an image, like a display. The display device 23 displays the image of the fertilized ovum 16, position information of the accommodation unit in which the fertilized ovum 16 is accommodated, information such as an image capturing date and time and a growth stage (growth stage code), and the like. In addition, an instruction for notifying processing or the like to be performed, to the user, is displayed on the display device 23, in addition to the image of the fertilized ovum 16, or various information items.

As illustrated in Fig. 5, the information processing apparatus 22 includes an image obtaining unit 222, an evaluation unit 223, a fertilized ovum database unit 224 as a storage unit, a display controller unit 225, an image capturing controller unit 226, and a determination unit 227. The information processing apparatus 22 controls the operation of each block in the observation system 2. In this embodiment, the information processing apparatus 22 controls the camera 25 such that the image of the fertilized ovum 16 is captured from one image capturing angle up to a certain growth stage, and after a certain growth stage, and the number of times of image capturing of the rotated fertilized ovum 16 is more than the number of times of image capturing so far. Thus, in the image capturing after a certain growth stage, the image of the fertilized ovum 16 is captured while rotating the fertilized ovum 16, and thus, the images of the fertilized ovum 16, captured from the plurality of angles, are obtained. In addition, the information processing apparatus 22 evaluates the fertilized ovum 16 on the basis of the image of the fertilized ovum. In this embodiment, a certain growth stage that is a timing when the image of the rotated fertilized ovum 16 is captured, is a growth stage of the complete blastocyst.

The information processing apparatus 22 includes hardware necessary for configuring a computer, such as a central processing unit (CPU), a read only memory (ROM), a random access memory (RAM), and a hard disk drive (HDD). For example, a personal computer (PC) is used as the information processing apparatus 22, but other arbitrary computers may be used.

The image obtaining unit 222, the evaluation unit 223, the fertilized ovum database unit 224, the display controller unit 225, the image capturing controller unit 226, and the determination unit 227 that are functional blocks of the information processing apparatus 22, are realized by allowing the CPU to execute a program stored in the ROM that is an example of a non-transitory computer readable recording medium by loading the program in the RAM. Then, according to the functional blocks, an image obtaining method according to the present technology is executed. The program, for example, is installed in the information processing apparatus 22 through various storage media. Alternatively, the installation of the program may be executed through the internet or the like.

The image obtaining unit 222 obtains image information captured by the camera 25, from the camera 25.

The evaluation unit 223 extracts a second feature amount of the fertilized ovum, on the basis of the image of the fertilized ovum 16, obtained by the camera 25, evaluates the growth stage of the fertilized ovum 16 with reference to the extracted second feature amount, and a first feature amount of each growth stage of the fertilized ovum 16, stored in advance in the fertilized ovum database unit 224, and applies a growth stage code described below to the fertilized ovum 16.

The feature amount is information of a feature portion of the image, and for example, is extracted on the basis of various growth stages of the fertilized ovum 16, such as the size or the shape, and the sphericity of the fertilized ovum, the number of segmentations (a segmentation rate), the form of each blastomere and a balance thereof, fragmentation, and the size or the shape of the ICM. In this embodiment, a feature amount to be extracted on the basis of the image of the fertilized ovum 16 to be observed, is set to the second feature amount, and a feature amount stored in advance, which is referred to the evaluation of the growth stage of the fertilized ovum 16, is set to the first feature amount. Specific feature extraction processing for extracting the feature amount is not limited, and arbitrary extraction processing may be used. In this embodiment, a machine learning algorithm is used.

For example, a machine learning algorithm using a neural network such as a recurrent neural network (RNN), a convolutional neural network (CNN), and multilayer perceptron (MLP), is used. In addition, an arbitrary machine learning algorithm for executing a supervised learning method, an unsupervised learning method, a semi-supervised learning method, a reinforcement learning method, or the like, may be used.

Here, the evaluation of the growth stage is applied to the image of the fertilized ovum 16 as the growth stage code. For example, the growth stage of the 1-cell period 1601 is set to a growth stage code 1, the growth stage from the 2-cell period 1602 to the 16-cell period 1605 is set to a growth stage code 2, the growth stage of the early morula 1606 is set to a growth stage code 3, the growth stage of the morula 1607 is set to a growth stage code 4, the growth stage of the early blastocyst 1608 is set to a growth stage code 5, the growth stage of the complete blastocyst 1609 is set to a growth stage code 6, the growth stage of the expanded blastocyst 1610 is set to a growth stage code 7, the growth stage of the hatched blastocyst 1611 is set to a growth stage code 8, and the growth stage of the expanded hatched blastocyst 1612 is set to a growth stage code 9.

The fertilized ovum database unit 224 stores the image of the fertilized ovum 16, captured by the camera 25, in each accommodation unit 15 in chronological order, along with the position information of the accommodation unit 15 in which the fertilized ovum 16 is accommodated, and information such as the image capturing date and time, an image capturing condition, the second feature amount of the fertilized ovum 16, extracted by the evaluation unit 223, and the growth stage (the growth stage code). In addition, a plurality of first feature amounts of the image of each of the growth stages of the fertilized ovum 16 are are stored in advance in the fertilized ovum database unit 224, as supervised data.

The display controller unit 225 allows the display device 23 to display the image of the fertilized ovum 16, and the position information of the accommodation unit in which the fertilized ovum 16 is accommodated, the image capturing date and time, the image capturing condition, the second feature amount of the fertilized ovum, the growth stage, and the like, in association with the image of the fertilized ovum 16.

In addition, in a case where the determination unit 227 determines that it is the fertilized ovum 16 is in a growth stage after the growth stage code 6, that is, in a growth stage after the complete blastocyst, the display controller unit 225 allows the display device 23 to display an instruction for notifying that the fertilized ovum 16 is in the growth stage after the complete blastocyst, and for rotating the fertilized ovum 16 with respect to the user. The user rotates the fertilized ovum 16 by using optical tweezers or a micropipette, a water flow of a micro-flow path, or the like, according to the instruction displayed on the display device 23. According to the start of the rotation of the fertilized ovum 16, the user inputs the start of the rotation of the fertilized ovum 16 to the information processing apparatus 22 by using the input device 29 such as a mouse. The rotation of the fertilized ovum using the water flow of the micro-flow path, will be described in a third embodiment described below. In the rotation using the water flow, the micro-flow path connected to each of the accommodation units of the culture vessel, is provided, and the accommodated fertilized ovum is rotated by the water flow from the micro-flow path.

The determination unit 227 determines whether or not the growth stage code applied by the evaluation unit 223 is after the growth stage code 6.

The image capturing controller unit 226 outputs a control signal for controlling the number of times of image capturing of the fertilized ovum 16, to the camera 25. The image capturing controller unit 226 allows the camera 25 to capture the image of the fertilized ovum 16 from one image capturing angle, with respect to the fertilized ovum 16 before the growth stage code 5 including up to the fertilized ovum 16 that is determined that the growth stage code is not after the growth stage code 6 by the determination unit 227, that is, growth stage of the morula of which the shape is comparatively symmetric. The image capturing controller unit 226 allows the camera 25 to capture the image of the fertilized ovum 16 in a rotation state such that the number of times of image capturing is more than the number of times of image capturing of the fertilized ovum 16 of the growth stage codes 1 to 5 so far, with respect to the fertilized ovum 16 that is determined that the growth stage code is after the growth stage code 6 by the determination unit 227, that is, the fertilized ovum of the growth stage after the complete blastocyst, in a shape having high asymmetry properties.

In addition, in a case where an input manipulation for starting the rotation of the fertilized ovum 16 is performed by the user, with respect to the fertilized ovum 16 that is determined that the growth stage code is after the growth stage code 6, the image capturing controller unit 226 allows the camera 25 to start the image capturing.

The image capturing angle is fixed, and the image capturing is performed a plurality of times while rotating the fertilized ovum 16, and thus, it is possible to obtain the images of the fertilized ovum 16, captured from a plurality of different angles.

In a case where the rotation of the fertilized ovum 16 is ended, the user performs an input manipulation for ending the rotation of the fertilized ovum by using the input device 29. The input manipulation for starting and ending a rotation operation, may be performed for each of the accommodation units 15, or may be performed for each of the culture vessels 1 or each of the dish holders 5.

Note that, here, a plurality of images are obtained while rotating the fertilized ovum 16, but the rotated fertilized ovum 16 may be obtained as a moving image, and a plurality of arbitrary images may be extracted the obtained moving image.

The image obtaining unit 222 obtains the image of the fertilized ovum 16, captured by the camera 25. The image obtaining unit 222 obtains the image of the fertilized ovum 16 of the growth stage codes 1 to 5 determined that the growth stage code is not after the growth stage code 6 by the determination unit 227, for example, captured from one image capturing angle, and obtains the image of the fertilized ovum 16 determined that the growth stage code is after the growth stage code 6 by the determination unit 227, captured from the plurality of angles.

### <Image Obtaining Method>

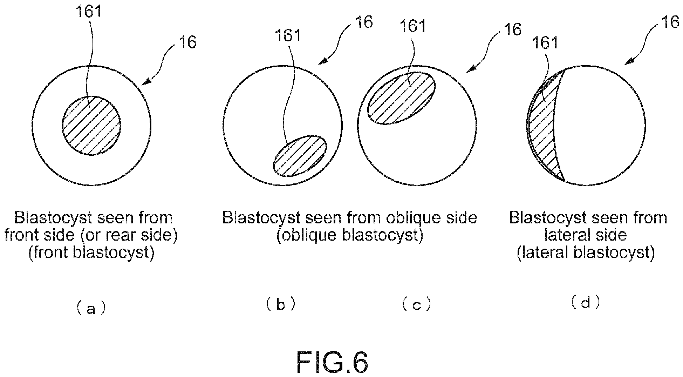

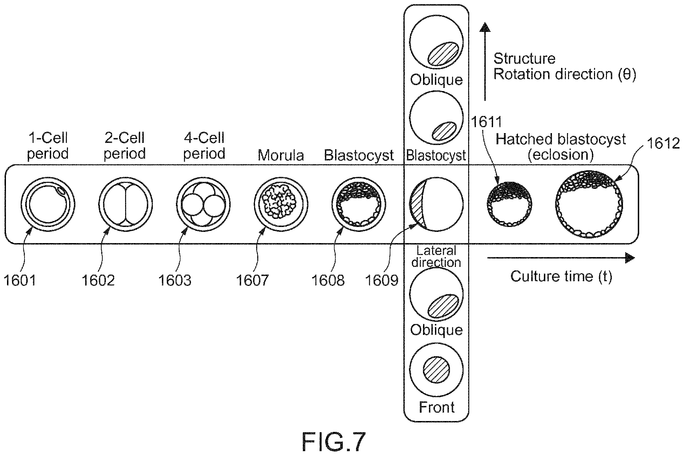

Next, an image obtaining method as the information processing method of the observation system 2 described above, will be described by using Fig. 4, and Fig. 6 to Fig. 9. Fig. 6 is a schematic view of the image of the fertilized ovum 16 in the growth stage after the complete blastocyst. Fig. 7 is a diagram illustrating an image obtained by the observation system 2.

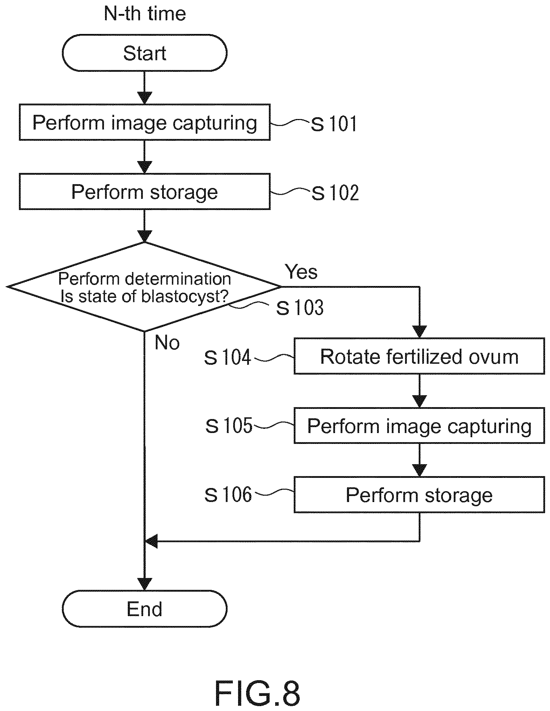

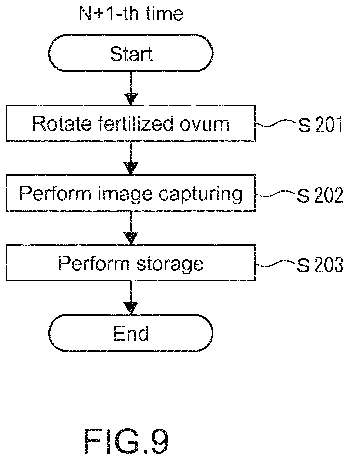

Fig. 8 and Fig. 9 are flowcharts of the image obtaining method. Fig. 8 is an image obtaining method performed with respect to the fertilized ovum 16 up to the growth stage code 6, and Fig. 9 is an image obtaining method performed with respect to the fertilized ovum 16 after the growth stage code 7. Here, the images of the fertilized ovum 16 in the culture vessel 1 provided in the observation apparatus 21, are sequentially captured one by one. Note that, in this embodiment, the images of the fertilized ovum 16 are sequentially captured one by one, but a plurality of images may be collectively captured. Hereinafter, the image obtaining method will be described according to the flow of Fig. 8 and Fig. 9.

The image of the fertilized ovum 16 is obtained in a state where the culture vessel 1 in which the fertilized ovum 16 is accommodated, is retained in the observation apparatus 21. Image obtaining processing of the fertilized ovum 16, is sequentially performed with respect to each of the fertilized ova 16 accommodated in each of the plurality of accommodation units 15 of the culture vessel 1. The image obtaining processing, for example, is automatically performed every 15 minutes, and the obtained image is stored in chronological order, along with the position information of the accommodation unit in which the fertilized ovum 16 is accommodated, and the information such as the image capturing date and time, the image capturing condition, the second feature amount of the fertilized ovum 16, and the growth stage, for each of the fertilized ova 16.

First, the fertilized ovum 16 of which the fertilization is confirmed is put into the accommodation unit 15 of the culture vessel 1 one by one, and then, the broth 18 is injected into the accommodation unit 15 and the region surrounded by the inner-wall 12, with a pipette. After that, the oil 17 is injected into the region surrounded by the inner-wall 12 to cover the broth 18.

Next, as illustrated in Fig. 4, the culture vessel 1 is horizontally placed on the stage 27 in the observation apparatus 21. At this time, a transparent lid (not illustrated) formed of the same material as that of the culture vessel 1 may be provided in the culture vessel 1, as necessary.

Next, light from the light source 24 is emitted from the lower portion of the culture vessel 1, and the image of the fertilized ovum 16 is captured by the camera 25 positioned in the upper portion of the culture vessel 1 (S101). The camera 25 is controlled by the image capturing controller unit 226 such that the image of the fertilized ovum 16 is captured from one observation (image capturing) angle.

The image captured by the camera 25, is obtained by the image obtaining unit 222. Image preprocessing such as the normalization of the image, the adjustment of the position of the fertilized ovum 16, and an emphasis filter of the shape, may be executed with respect to the obtained image of the fertilized ovum 16.

Next, the second feature amount of the fertilized ovum 16 is extracted by the evaluation unit 223, on the basis of the image of the fertilized ovum 16, obtained by the image obtaining unit 222. Further, the growth stage of the fertilized ovum 16 is evaluated by the evaluation unit 223 by referring to the extracted second feature amount, and the first feature amount of the fertilized ovum 16 in each of the growth stages, stored in advance in the fertilized ovum database unit 224, and the growth stage code is applied to the fertilized ovum 16.

The image of the fertilized ovum 16, the position information of the accommodation unit in which the fertilized ovum 16 is accommodated, the image capturing date and time, the image capturing condition, the second feature amount, and the growth stage (the growth stage code) are stored in association with each other, by the fertilized ovum database unit 224 (S102).

Next, whether or not the growth stage of the fertilized ovum 16, applied by the evaluation unit 223, is the state of the blastocyst, in this embodiment, a state after the complete blastocyst, that is, the growth stage code is after the growth stage code 6, is determined by the determination unit 227 (S103).

In a case where it is determined as No in S103, the image capturing is ended. In a case where it is determined as YES in S103, the display device 23 displays that the fertilized ovum 16 is in the growth stage of the complete blastocyst, and displays an instruction for rotating the fertilized ovum 16, by the display controller unit 225 (S104).

The instruction of the rotation of the fertilized ovum 16, displayed on the display device 23, is confirmed by the user. When the instruction of the rotation of the fertilized ovum 16, is displayed on the display device 23, the instruction of the rotation may be notified to the user by generating a sound.

An input manipulation for notifying the start of the rotation is performed from the input device 29 by the user immediately before the start of the rotation of the fertilized ovum 16. In a case where the input manipulation for starting the rotation is performed by the user, the image capturing controller unit 226 controls the camera 25 such that the number of times of image capturing of the rotated fertilized ovum 16 is more than the number of times of image capturing of the fertilized ovum 16 of the growth stage codes 1 to 5 so far. Hereinafter, the number of times of image capturing of the fertilized ovum 16 after the growth stage code 6, is more than the number of times of image capturing of the fertilized ovum 16 up to the growth stage code 5.

The rotation of the fertilized ovum 16 is started by the user with the optical tweezers, the micropipette, or the like. The image of the fertilized ovum 16 is captured by the camera 25 a plurality of times while the fertilized ovum 16 is rotated (S105). The image captured by the camera 25, is obtained by the image obtaining unit 222. The rotation of the fertilized ovum 16 according to the user, is ended, and an input manipulation for notifying the end of the rotation is performed from the input device 29 by the user, and thus, the image capturing of the fertilized ovum 16 is ended. The image of the rotated fertilized ovum 16 is captured, and thus, the image of the fertilized ovum 16 is captured from the plurality of angles.

The plurality of images of the fertilized ovum 16, captured from the plurality of angles, the position information of the accommodation unit in which the fertilized ovum 16 is accommodated, and the information such as the image capturing date and time, the image capturing condition, and the second feature amount and the growth stage (the growth stage code) of the fertilized ovum, are stored in association with each other, by the fertilized ovum database unit 224 (S106).

In a case where the N-th image is obtained when it is determined as YES in S103, the N+1-th image and the subsequence are obtained according to the flowchart illustrated in Fig. 9. In addition, in a case where it is determined as NO in S103, the next image is obtained according to the flowchart illustrated in Fig. 8, and the image is obtained according to the flowchart illustrated in Fig. 8, until it is determined as YES in S103.

Next, an obtaining method of the image subsequent to the N+1-th image will be described by using Fig. 9.

In a case where image processing is started, the display device 23 displays that the fertilized ovum 16 is in the growth stage of the blastocyst, and displays the instruction for rotating the fertilized ovum 16, by the display controller unit 225 (S201).

The user confirms the instruction for rotating the fertilized ovum 16, displayed on the display device 23, and thus, the fertilized ovum 16 is rotated by using the optical tweezers or the micropipette, water flow control of the micro-flow path provided in the culture vessel, or the like (S201).

The user performs the input manipulation for notifying the start of the rotation from the input device 29 immediately before the start of the rotation of the fertilized ovum 16. In a case where the input manipulation for starting the rotation start is performed by the user, the camera 25 is controlled by the image capturing controller unit 226 such that the number of times of image capturing of the rotated fertilized ovum 16, is more than the number of times of image capturing of the fertilized ovum 16 before the growth stage code 5.

The camera 25 performs the image capturing such that the number of times of image capturing of the fertilized ovum 16 is more than the number of times of image capturing of the fertilized ovum 16 up to the growth stage code 5 while the user rotates the fertilized ovum 16 (S202). The image captured by the camera 25, is obtained by the image obtaining unit 222. The rotation of the fertilized ovum 16 according to the user, is ended, the input manipulation for notifying the end of the rotation is performed from the input device 29 by the user, and thus, the image capturing of the fertilized ovum 16 is ended.

Next, the second feature amount of the fertilized ovum 16 is extracted on the basis of the image of the fertilized ovum 16, obtained by the image obtaining unit 222, the growth stage of the fertilized ovum 16 is evaluated, and the growth stage code is applied to the fertilized ovum 16, by the evaluation unit 223.

The plurality of images of the fertilized ovum 16, captured from the plurality of angles, the position information of the accommodation unit 15 in which the fertilized ovum 16 is accommodated, and the information such as the image capturing date and time, the image capturing condition, and the second feature amount and the growth stage (the growth stage code) of the fertilized ovum are stored in association with each other, by the fertilized ovum database unit 224 (S203).

As described above, the fertilized ovum having high structural asymmetry properties, after the growth stage of the blastocyst, is rotated, and the image capturing is performed a plurality of times, and thus, it is possible to obtain the images of the fertilized ovum, captured from the plurality of angles. Accordingly, in the evaluation of the fertilized ovum, it is possible to comprehensively evaluate the images of the cell, captured from various angles, and to perform the evaluation with a high accuracy.

The image of the fertilized ovum 16 is captured at an arbitrary image capturing interval by using the image obtaining method described above, the data of the fertilized ovum 16 as illustrated in Fig. 7 can be obtained in chronological order. Note that, in Fig. 7, for the sake of convenience, a few images of the fertilized ovum are illustrated.

In Fig. 7, the images of the fertilized ovum 16 in several growth stages, are arranged in a lateral direction in chronological order. In Fig. 7, the fertilized ova 16 in each of the growth stages such as the 1-cell period 1601 of the growth stage code 1, the 2-cell period 1602 of the growth stage code 2, the 4-cell period 1603, the morula 1607 of the growth stage code 4, the early blastocyst 1608 of the growth stage code 5, the complete blastocyst 1609 of the growth stage code 6, the hatched blastocyst 1611 of the growth stage code 8, the expanded hatched blastocyst 1612 of the growth stage code 9, are sequentially illustrated from the left. In Fig. 7, it is illustrated that the images of the fertilized ovum 16, captured from the plurality of angles, are arranged in a vertical direction, only in the fertilized ovum 16 of the complete blastocyst 1609 of the growth stage code 6, and the same image capturing is performed with respect to the fertilized ovum 16 in certain growth stage codes 7 to 9 in the subsequent growth stage, from the plurality of angles, and thus, the images of the fertilized ovum 16 are obtained.

Here, as illustrated in Figs. 3(i), 3(j), 3(k), and 3(l), the ratio of the blastocoele in the fertilized ovum 16 in a growth stage after the complete blastocyst 1609, increases, and the ICM 161 biasedly exists on the end in the spherical fertilized ovum 16.

Fig. 6 is a schematic view of the images of the fertilized ovum 16 in the growth stage after the complete blastocyst 1609, captured from the plurality of angles. The obtained image of the fertilized ovum 16 after the growth stage code 6, may be stored by being sorted into three image patterns of a front or rear pattern, an oblique pattern, and a lateral pattern, according to the shape and the position of the ICM 161. The front or rear pattern, the oblique pattern, and the lateral pattern indicate an observation (an image capturing) direction of the fertilized ovum at the time of observing the fertilized ovum 16 on the basis of the front.

A state in which the ICM 161 is positioned on the front center of the fertilized ovum 16, is set to the front, the image of the fertilized ovum 16, captured from the front or the rear, as illustrated in Fig. 6(a), is an image in which an approximately circular ICM 161 is positioned in the center portion of the fertilized ovum 16, and the image is set to a front or rear image.

A state in which the ICM 161 is positioned on the front side of the fertilized ovum, is set to the front, and the image of the fertilized ovum 16, captured from the lateral direction, as illustrated in Fig. 6(d), is an image in which an approximately minor arcuate ICM 161 is positioned on the end in the fertilized ovum 16. The image is a lateral image.

A state in which the ICM 161 is positioned on the front side of the fertilized ovum 16, is set to the front, and a part of an image in a case where the image of the fertilized ovum 16 is captured from an oblique direction other than the lateral side and the rear side, as illustrated in Fig. 6(b) or Fig. 6(c), is an image in which the ICM 161 is positioned on the end in the fertilized ovum 16, in an approximately elliptical shape. The image is set to an oblique image.

In Fig. 7, it is illustrated that images of the complete blastocyst 1609, captured from a plurality of image capturing angles, are arranged in the vertical direction, the images respectively indicate images of the fertilized ovum 16 of the complete blastocyst, captured from the oblique direction, the oblique direction, the lateral direction, the oblique direction, and the front side, sequentially from the upper side.

Thus, the images of the fertilized ovum 16 after the growth stage code 6, captured from the plurality of image capturing angles, may be fertilized ovum database unit 224 by being sorted into the front or rear image, the oblique image, and the lateral image, according to the shape and the position of the ICM 161.

In addition, in this embodiment, it is controlled such that the number of times of image capturing of the fertilized ovum 16 before the growth stage code 5 is less than the number of times of image capturing of the fertilized ovum 16 after the growth stage code 6. Accordingly, it is possible to reduce an irradiation dose of light with respect to the fertilized ovum 16, necessary for the image capturing, rather than increasing the number of times of image capturing of the fertilized ovum 16 before the growth stage code 5, and to reduce the damage of the fertilized ovum 16 due to light.

### (Second Embodiment)

In the first embodiment, the rotation of the fertilized ovum 16 is performed by being manually operated with a micropipette or by controlling the water flow from the micro-flow path provided in the culture vessel, but a rotation operation may be automatized by rotating the fertilized ovum 16 with an apparatus. The rotation of the fertilized ovum 16 according to the apparatus, is controlled by the information processing apparatus. Hereinafter, a case in which a vibration apparatus vibrating the culture vessel 1 is provided as the apparatus rotating the fertilized ovum 16, will be described as a second embodiment. Configurations different from the configurations of the first embodiment, will be mainly described, the same reference numerals will be applied to the same configurations as those of the first embodiment, and there is a case where the description thereof will be emitted.

### <Observation System>

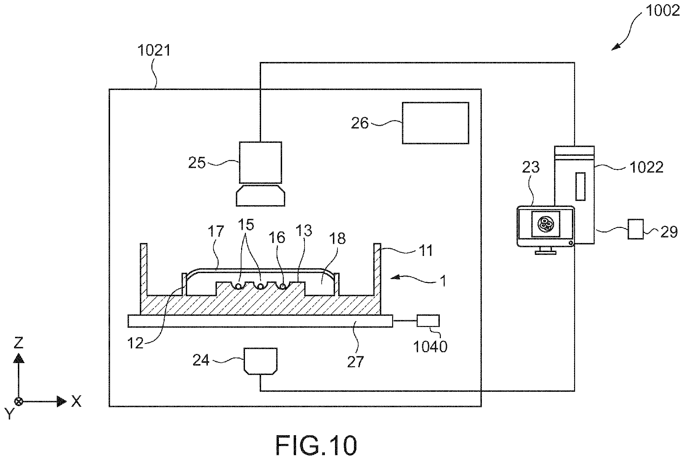

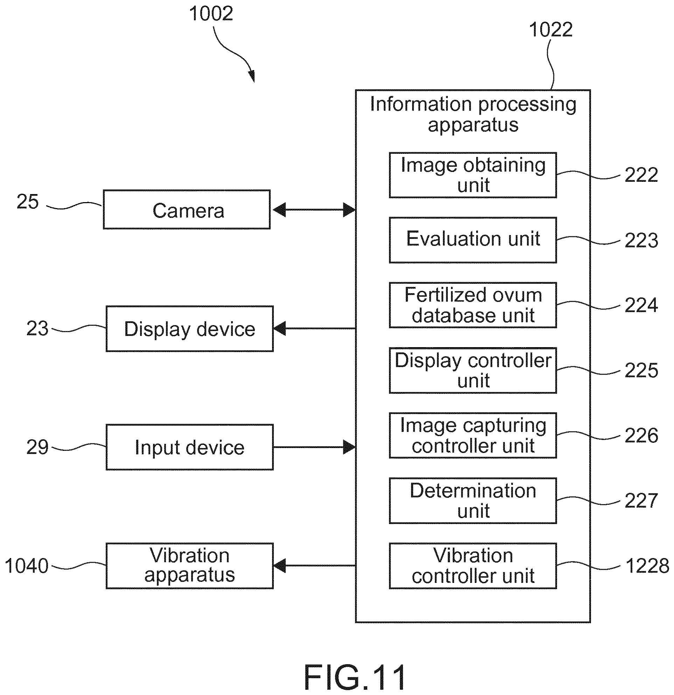

An observation system of the second embodiment will be described by using Fig. 10 and Fig. 11. Fig. 10 is a schematic view illustrating the observation system. Fig. 11 is a block diagram illustrating the configuration of the observation system.

An observation system 1002 includes an observation apparatus 1021, an information processing apparatus 1022, the display device 23, the input device 29, and a vibration apparatus 1040. The vibration apparatus 1040 as a rotation mechanism, vibrates the stage 27, and applies a vibration to the culture vessel 1 that is placed on the stage 27, and thus, rotates the fertilized ovum 16 accommodated in the accommodation unit 15 of the culture vessel 1, and is arranged in the observation apparatus 1021.

The information processing apparatus 1022 includes an image obtaining unit 222, an evaluation unit 223, a fertilized ovum database unit 224, a display controller unit 225, an image capturing controller unit 226, a determination unit 227, and a vibration controller unit 1228. The information processing apparatus 1022 controls the operation of each block in the observation system 1002. In this embodiment, the information processing apparatus 1022 controls such that the camera 25 captures the image of the fertilized ovum 16 from one image capturing angle, up to the growth stage of the early blastocyst, and in the growth stage after the complete blastocyst, the number of times of image capturing of the rotated fertilized ovum 16 is more than the number of times of image capturing so far. In the image capturing of the growth stage after the complete blastocyst, the image capturing is performed from one image capturing angle a plurality of times while rotating the fertilized ovum 16, and thus, the images of the fertilized ovum 16, captured from the plurality of angles, are obtained. In addition, the growth stage of the fertilized ovum 16 is evaluated on the basis of the image of the fertilized ovum, by the information processing apparatus 1022. In addition, the presence or absence and a timing for applying the vibration to the culture vessel 1, are controlled by the information processing apparatus 1022.

The image obtaining unit 222, the evaluation unit 223, the fertilized ovum database unit 224, the display controller unit 225, the image capturing controller unit 226, the determination unit 227, and the vibration controller unit 1228 that are functional blocks of the information processing apparatus 1022, are realized by allowing the CPU to execute a program stored in the ROM that is an example of a non-transitory computer readable recording medium by loading the program in the RAM. Then, according to the functional blocks, an image obtaining method according to the present technology is executed.

The evaluation unit 223 extracts the second feature amount of the fertilized ovum, on the basis of the image of the fertilized ovum 16, obtained by the camera 25, evaluates the growth stage of the fertilized ovum 16 by referring to the extracted second feature amount, and the first feature amount of the fertilized ovum 16 in each of the growth stages, stored in advance in the fertilized ovum database unit 224, and applies the growth stage code to the fertilized ovum 16.

The determination unit 227 determines whether or not the growth stage code applied by the evaluation unit 223, is after the growth stage code 6.

The vibration controller unit 1228 controls the presence or absence of the operation of the vibration apparatus 1040, according to a determination result of the determination unit 227. The vibration controller unit 1228 does not operate the vibration apparatus 1040 at the time of capturing the image of the fertilized ovum 16 determined that the growth stage code is not after the growth stage code 6 by the determination unit 227. The vibration controller unit 1228 operates the vibration apparatus 1040 at the time of capturing the image of the fertilized ovum 16 determined that the growth stage code is after the growth stage code 6 by the determination unit 227. According to the operation of the vibration apparatus 1040, the vibration is applied to the culture vessel 1 placed on the stage 27. By applying the vibration, the fertilized ovum 16 accommodated in the culture vessel 1, is vibrated and rotated.

### <Image Obtaining Method>

Next, an image obtaining method as the information processing method of the observation system 1002 described above, will be described, the image obtaining method is different from the image obtaining method of the first embodiment, in that the rotation of the fertilized ovum is not performed by a manual operation, but is performed by using a vibration apparatus, and hereinafter, the image obtaining method will be described by using Fig. 8 and Fig. 9 that are the same flowcharts as those of the first embodiment.

As with the first embodiment, the culture vessel 1 in which the fertilized ovum 16 is accommodated, is placed on the stage 27 in the observation apparatus 1021. Next, light from the light source 24, is emitted from the lower portion of the culture vessel 1, and the image of the fertilized ovum 16 is captured by the camera 25 positioned in the upper portion of the culture vessel 1 (S101).

The image obtained by the camera 25, is obtained by the image obtaining unit 222. Image preprocessing such as the normalization of the image, the adjustment of the position of the fertilized ovum 16, and the emphasis filter of the shape, may be executed with respect to the obtained image of the fertilized ovum 16.

Next, the second feature amount of the fertilized ovum 16 is extracted on the basis of the image of the fertilized ovum 16, obtained by the image obtaining unit 222, by the evaluation unit 223. Further, the growth stage of the fertilized ovum 16 is evaluated by referring to the extracted second feature amount, and the first feature amount of the fertilized ovum 16 in each of the growth stages, stored in advance in the fertilized ovum database unit 224, and the growth stage code is applied to the fertilized ovum 16, by the evaluation unit 223.

The image of the fertilized ovum 16, the position information of the accommodation unit in which the fertilized ovum 16 is accommodated, and the information such as the image capturing date and time, the image capturing condition, the second feature amount, and the growth stage (the growth stage code) are stored in association with each other, by the fertilized ovum database unit 224 (S102).

Next, whether or not the growth stage code is after the growth stage code 6, is determined by the determination unit 227 (S103). In a case where it is determined as No in S103, the image capturing is ended.

In a case where it is determined as YES in S103, the vibration apparatus 1040 is operated, the stage 27 is vibrated, and the fertilized ovum 16 is rotated, by the vibration controller unit 1228 (S104). In a case where it is determined as YES in S103, the image capturing controller unit 226 controls the camera 25 such that the number of times of image capturing of the rotated fertilized ovum 16 is more than the number of times of image capturing of the fertilized ovum 16 of the growth stage codes 1 to 5 so far. Hereinafter, the number of times of image capturing of the fertilized ovum 16 after the growth stage code 6, is more than the number of times of image capturing up to the growth stage code 5.

The camera 25 captures the image of the fertilized ovum 16 a plurality of times while the vibration apparatus 1040 is operated, and the fertilized ovum 16 is rotated (S105). The image captured by the camera 25, is obtained by the image obtaining unit 222.

The captured image at the time of rotating the fertilized ovum 16, the position information of the accommodation unit in which the fertilized ovum 16 is accommodated, and the information such as the image capturing date and time, the image capturing condition, and the second feature amount and the growth stage (the growth stage code) of the fertilized ovum, are stored in association with each other, by the fertilized ovum database unit 224 (S106).

In a case where the N-th image is obtained when it is determined as YES in S103, the N+1-th image and the subsequence are obtained according to the flowchart illustrated in Fig. 9. In addition, in a case where it is determined as NO in S103, the next image is obtained according to the flowchart illustrated in Fig. 8, and the image is obtained according to the flowchart illustrated in Fig. 8, until it is determined as YES in S103.

Next, an obtaining method of the image subsequent to the N+1-th image will be described by using Fig. 9.

In a case where the image processing is started, the vibration apparatus 1040 is operated, the stage 27 is vibrated, and the fertilized ovum 16 is rotated, by the vibration controller unit 1228 (S201). The image of the fertilized ovum 16 is captured such that the number of times of image capturing is more than the number of times of image capturing up to the growth stage code 5, by the image capturing controller unit 226 (S202). The image captured by the camera 25, is obtained by the image obtaining unit 222.

Next, the second feature amount of the fertilized ovum 16 is extracted on the basis of the image of the fertilized ovum 16, obtained by the image obtaining unit 222, the growth stage of the fertilized ovum 16 is evaluated, and the growth stage code is applied, by the evaluation unit 223. The plurality of images of the fertilized ovum 16, captured from the image plurality of angles, the position information of the accommodation unit in which the fertilized ovum 16 is accommodated, and the information such as the image capturing date and time, the image capturing condition, and the second feature amount and the growth stage (the growth stage code) of the fertilized ovum 16, are stored in association with each other, by the fertilized ovum database unit 224 (S203).

As described above, in a state where the fertilized ovum after the growth stage of the blastocyst, having high structural asymmetry properties, is rotated, the image capturing is performed a plurality of times, and thus, it is possible to obtain the images of the fertilized ovum, captured from the plurality of angles. Accordingly, it is possible to more accurately perform state evaluation of the fertilized ovum, and to increase an evaluation accuracy. In addition, in this embodiment, the rotation of the fertilized ovum is not performed by the manual operation, but is performed by using the apparatus, and the rotation of the fertilized ovum is controlled and automatized by the information processing apparatus 1022, and thus, it is possible to observe a large amount of fertilized ova. For example, in a case of observing the culture vessel in which 25 fertilized ova can be accommodated, by being retained in six dish holders, 150 fertilized ova can be simultaneously cultured and observed. In the observation of such a large amount of fertilized ova, an observation system in which an image is obtained by automatically rotating the fertilized ovum, as with this embodiment, is effective.

### (Third Embodiment)

In a third embodiment, the fertilized ovum 16 is rotated by using the apparatus, as with the second embodiment. In the second embodiment, the vibration is applied to the culture vessel, and the fertilized ovum 16 is rotated, by using the vibration apparatus, but in the third embodiment, a fluid is jetted (injected) into the broth of the accommodation unit of the culture vessel, and thus, a flow is generated in the broth in the accommodation unit, and the fertilized ovum 16 is rotated.

The third embodiment is mainly different from the second embodiment, in that the culture vessel including the rotation mechanism rotating the fertilized ovum not by the vibration apparatus but by generating the flow in the broth, is used, and will be described below. Configurations different from the configurations of the second embodiment, will be mainly described, the same reference numerals will be applied to the same configurations as those of the second embodiment, and there is a case where the description thereof will be emitted.

### <Observation System>

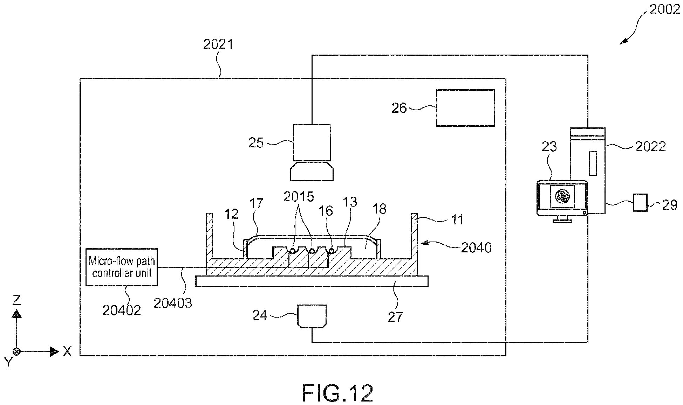

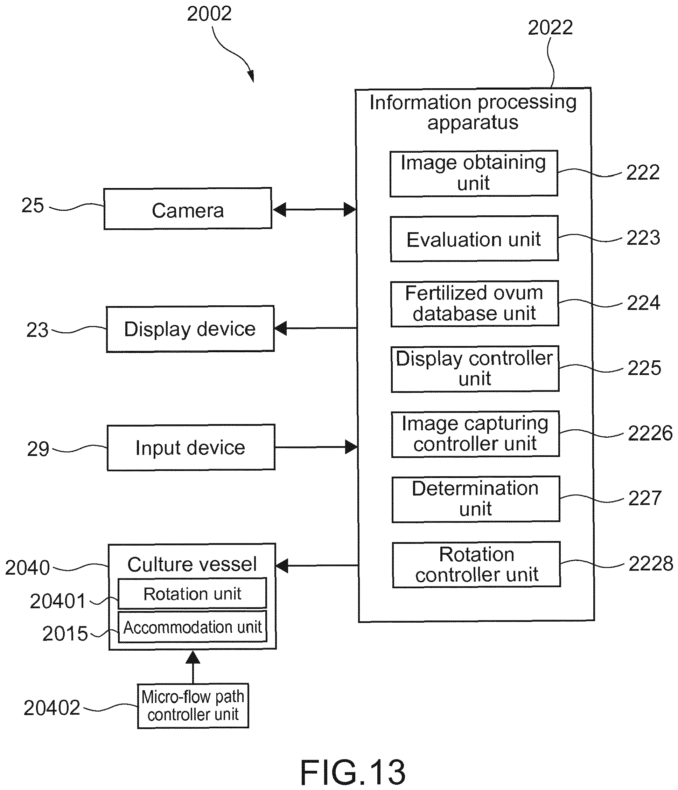

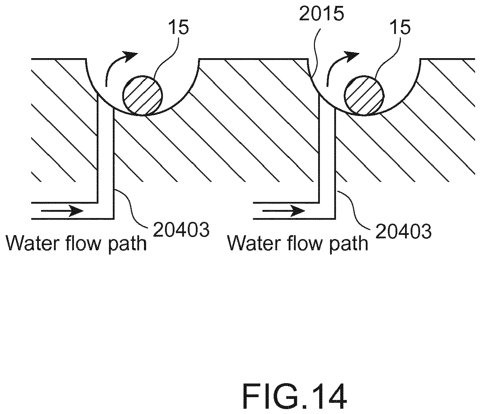

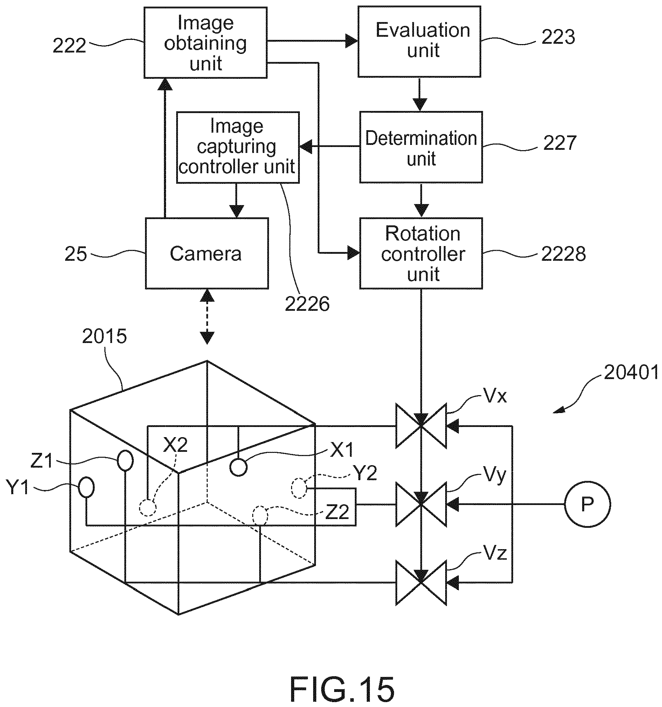

An observation system of the third embodiment will be described by using Fig. 12, Fig. 13, Fig. 14, and Fig. 15. Fig. 12 is a schematic view illustrating the configuration of the observation system. Fig. 13 is a block diagram illustrating the configuration of the observation system. Fig. 14 is a partially enlarged view of the vicinity of the accommodation unit of the culture vessel. Fig. 15 is a diagram illustrating a configuration in the vicinity of the rotation unit of the observation system.

An observation system 2002 includes an observation apparatus 2021, an information processing apparatus 2022, the display device 23, and the input device 29. In the observation apparatus 21, the light source 24, the camera 25 as an image capturing unit, the temperature·humidity·gas controller unit 26, the stage 27, and a culture vessel 2040 including a rotation mechanism, are arranged.

The culture vessel 2040 including the rotation mechanism, includes a rotation unit 20401 as a rotation mechanism, an accommodation unit 2015 accommodating the fertilized ovum 16, and a micro-flow path (a water flow path) 20403. The rotation unit 20401 is controls by a micro-flow path controller unit 20402. The rotation unit 20401 generates a flow in the broth 18 in the accommodation unit 2015 accommodating the fertilized ovum 16, and rotates the fertilized ovum 16. The micro-flow path controller unit 20402 controls the jet of a fluid into the accommodation unit 2015, and generates a flow in the broth 18 by jetting the fluid. The water flow path 20403 is a fluid flow path that is connected to each accommodation unit 2015, and supplies the fluid into each of the accommodation units 2015.

As with the accommodation unit 15 of the culture vessel 1 of the embodiments described above, the accommodation unit 2015 is capable of accommodating a liquid, and of holding one cell in a constant position while accommodating the cell in the liquid. The "liquid" is typically a broth suitable for culturing a cell, and here, will be described as a broth.

The rotation unit 20401 includes a pump P, an X-axis rotation valve Vx, a Y-axis rotation valve Vy, a Z-axis rotation valve Vz, a first X-axis jet port X1 (a first output port), a second X-axis jet port X2 (a second output port), a first Y-axis jet port Y1 (a first output port), a second Y-axis jet port Y2 (a second output port), a first Z-axis jet port Z1 (a first output port), and a second Z-axis jet port Z2 (a second output port). Here, an X-axis, a Y-axis, and a Z-axis indicate three orthogonal axes, and do not indicate a horizontal direction and a vertical direction.

The first X-axis jet port X1, the second X-axis jet port X2, the first Y-axis jet port Y1, the second Y-axis jet port Y2, the first Z-axis jet port Z1, and the second Z-axis jet port Z2 are formed on an inner-wall surface of the accommodation unit 2015 (in a case where there are a plurality of accommodation units 2015, the jet ports are independently formed with respect to all of the accommodation units 2015, respectively). The first X-axis jet port X1, the second X-axis jet port X2, the first Y-axis jet port Y1, the second Y-axis jet port Y2, the first Z-axis jet port Z1, and the second Z-axis jet port Z2 respectively jet (inject) the fluid into the broth in the accommodation unit 2015, and thus, generate a flow in the broth in the accommodation unit 2015. The "fluid" is typically the same liquid as the broth in the accommodation unit 2015, but may be a liquid different from the broth in the accommodation unit 2015, or gas.

The pump P is connected to each of the first X-axis jet port X1, the second X-axis jet port X2, the first Y-axis jet port Y1, the second Y-axis jet port Y2, the first Z-axis jet port Z1, and the second Z-axis jet port Z2, through the flow path, and supplies the broth to the jet ports. A part of each flow path (a portion not on the pump side but on the jet port side) is formed in a wall surface of the accommodation unit 2015 (in a case where there are a plurality of accommodation units 110, the flow paths are independently formed with respect to all of the accommodation units 2015, respectively).

The X-axis rotation valve Vx is provided in the flow path connecting the pump P, and the first X-axis jet port X1 and the second X-axis jet port X2, together. The Y-axis rotation valve Vy is provided in the flow path connecting the pump P, and the first Y-axis jet port Y1 and the second Y-axis jet port Y2, together. The Z-axis rotation valve Vz is provided in the flow path connecting the pump P, and the first Z-axis jet port Z1 and the second Z-axis jet port Z2, together.

The micro-flow path controller unit 20402 controls a jet speed and a jet volume of the broth jetted from each of the jet ports of the rotation unit 20401, on the basis of a control signal transmitted from the rotation controller unit 2228 described below.

The information processing apparatus 2022 includes the image obtaining unit 222, the evaluation unit 223, the fertilized ovum database unit 224, the display controller unit 225, an image capturing controller unit 2226, the determination unit 227, and a rotation controller unit 2228.

The information processing apparatus 2022 controls the operation of each block in the observation system 2002. In this embodiment, the number of times of image capturing of the camera 25 is controlled, and the image capturing of the camera 25 is controlled such that the image of the fertilized ovum 16 is captured from one angle up to a certain growth stage, and the number of times of image capturing of the rotated fertilized ovum 16 after a certain growth stage is more than the number of times of image capturing so far, by the information processing apparatus 2022. In addition, the growth stage of the fertilized ovum 16 is evaluated on the basis of the image of the fertilized ovum, by the information processing apparatus 2022. In addition, the operation of the rotation unit 20401 is controlled, and the presence or absence and the timing for applying the rotation of the fertilized ovum 16, are controlled, by the information processing apparatus 2022.

The image obtaining unit 222, the evaluation unit 223, the fertilized ovum database unit 224, the display controller unit 225, the image capturing controller unit 2226, the determination unit 227, and the rotation controller unit 2228 that are functional blocks of the information processing apparatus 2022, are realized by allowing the CPU to execute a program stored in the ROM that is an example non-transitory computer readable recording medium by loading the program in the RAM. Then, according to the functional blocks, an image obtaining method according to the present technology is executed.

The evaluation unit 223 extracts the second feature amount of the fertilized ovum, on the basis of the image of the fertilized ovum 16, obtained by the camera 25, evaluates the growth stage of the fertilized ovum 16 by referring to the extracted second feature amount, and the first feature amount of the fertilized ovum 16 in each of the growth stages, stored in advance in the fertilized ovum database unit 224, and applies the growth stage code described below to the fertilized ovum 16.

The determination unit 227 determines whether or not the growth stage of the fertilized ovum 16, evaluated by the evaluation unit 223, is the complete blastocyst, and specifically, whether or not the growth stage code is after the growth stage code 6.

The image capturing controller unit 2226 performs the image capturing of the fertilized ovum 16 such that the image of the fertilized ovum 16 determined that the growth stage code is not after the growth stage code 6 by the determination unit 227, is captured form one image capturing angle. The image capturing controller unit 2226 fixes the image capturing angle of the camera 25, with respect to the fertilized ovum 16 determined that the growth stage code is after the growth stage code 6 by the determination unit 227, and controls the camera 25 such that the number of times of image capturing of the rotated fertilized ovum 16 is more than the number of times of image capturing of the fertilized ovum 16 determined that the growth stage code is not the growth stage code 6, so far.

In addition, the image capturing controller unit 2226 controls the camera 25 such that the camera 25 captures the image of the fertilized ovum 16 determined that the growth stage code is after the growth stage code 6, in a state where the fertilized ovum 16 remains still immediately before the fertilized ovum 16 is rotated.

The rotation controller unit 2228 controls the presence or absence of the operation of the rotation unit 20401, according to a determination result of the determination unit 227, in other words, the growth stage code of the fertilized ovum 16, applied by the evaluation unit 223. The rotation controller unit 2228 transmits the control signal to the micro-flow path controller unit 20402 such that the rotation unit 20401 is not operated at the time of capturing the image of the fertilized ovum 16 determined that the growth stage code is not after the growth stage code 6 by the determination unit 227, and the rotation unit 20401 is operated at the time of capturing the image of the fertilized ovum 16 determined that the growth stage code is after the growth stage code 6.

In a case where the operation of the rotation unit 20401 is started on the basis of the control signal transmitted to the micro-flow path controller unit 20402 from the rotation controller unit 2228, the fluid is jetted into the accommodation unit 2015, a flow is generated in the broth 18 in the accommodation unit 2015, and the fertilized ovum 16 is rotated.

The rotation controller unit 2228 is capable of controlling the rotation of the fertilized ovum 16 such that an image of a desired fertilized ovum 16 is obtained. In this embodiment, as illustrated in Fig. 6, the image of a desired fertilized ovum 16 is three images of an image in which the ICM 161 is positioned in the center portion of the fertilized ovum 16 in the fertilized ovum in the growth stage after the complete blastocyst (referred to as the front or rear image in Fig. 6(a)), an image in which an approximately minor arcuate ICM 161 is positioned on the end in an approximately circular fertilized ovum 16 (referred to as the lateral image in Fig. 6(d)), and an image in which an approximately elliptical ICM 161 is positioned on the end in the fertilized ovum 16 (referred to as the oblique image in Figs. 6(b) and 6(c)).

The rotation controller unit 2228 determines the shape and the position of the ICM 161 according to image recognition such as edge detection, on the basis of the image of the fertilized ovum 16, captured immediately before the fertilized ovum 16 is rotated. On the basis of this, the rotation controller unit 2228 calculates a rotation direction and a rotation amount of the fertilized ovum 16 in order to position the ICM 161 in the center portion of the fertilized ovum 16. The fertilized ovum 16 is rotated by the rotation unit 20401, according to the calculated rotation direction and rotation amount. Similarly, the rotation controller unit 2228 calculates the rotation direction and the rotation amount of the fertilized ovum in order to position the approximately minor arcuate ICM 161 is on the end of the fertilized ovum 16, generates a control signal based on a calculation result, and transmits the control signal to the micro-flow path controller unit 20402. The micro-flow path controller unit 20402 controls the rotation unit 20401 on the basis of the control signal, and rotates the fertilized ovum 16. Further, similarly, the rotation controller unit 2228 calculates the rotation direction and the rotation amount of the fertilized ovum 16 in order to position the approximately elliptical ICM 161 on the end of the fertilized ovum 16, and rotates the fertilized ovum 16 by the rotation unit 20401, on the basis of the calculation result.

The micro-flow path controller unit 20402 independently controls the flows of the broth, generated by each of the jet ports X1, X2, Y1, Y2, Z1, and Z2 of the rotation unit 20401, on the basis of the control signal from the rotation controller unit 2228, and controls the rotation direction and the rotation amount of the fertilized ovum 16. Specifically, the micro-flow path controller unit 20402 controls the X-axis rotation valve Vx such that the X-axis rotation valve Vx is opened and closed, on the basis of the control signal from the rotation controller unit 2228, and controls the jet speed and the jet volume of the broth jetted from the first X-axis jet port X1 and the second X-axis jet port X2. The micro-flow path controller unit 20402 controls the Y-axis rotation valve Vy such that the Y-axis rotation valve Vy is opened and closed, on the basis of the control signal from the rotation controller unit 2228, and controls the jet speed and the jet volume of the broth jetted from the first Y-axis jet port Y1 and the second Y-axis jet port Y2. The micro-flow path controller unit 20402 controls the Z-axis rotation valve Vz such that the Z-axis rotation valve Vz is opened and closed, on the basis of the control signal from the rotation controller unit 2228, and controls the jet speed and the jet volume of the broth jetted from the first Z-axis jet port Z1 and the second Z-axis jet port Z2.

Thus, in this embodiment, it is possible to rotate the fertilized ovum 16 such that the ICM 161 is positioned in a desired position in the fertilized ovum 16, by controlling the rotation direction and the rotation amount of the fertilized ovum 16, and thus, it is possible to efficiently obtain a desired image.

### <Image Obtaining Method>

Next, an image obtaining method of the observation system 2002 described above, will be described. Hereinafter, the image obtaining method will be described by using Fig. 12, and Fig. 8 and Fig. 9 that are the same flowcharts as those of the first embodiment.

The culture vessel 2040 in which the fertilized ovum 16 is accommodated in the accommodation unit 2015, is horizontally placed on the stage 27 in the observation apparatus 2021. Next, light from the light source 24 is emitted from the lower portion of the culture vessel 2040, and the image of the fertilized ovum 16 is captured by the camera 25 positioned in the upper portion of the culture vessel 2040 (S101). The camera 25 is controlled by the image capturing controller unit 2226 such that the image of the fertilized ovum 16 is captured from one angle.

The image captured by the camera 25, is obtained by the image obtaining unit 222. Next, the second feature amount of the fertilized ovum 16 is extracted on the basis of the image of the fertilized ovum 16, obtained by the image obtaining unit 222, the growth stage of the fertilized ovum 16 is evaluated from the extracted second feature amount, and the growth stage code is applied to the fertilized ovum 16, by the evaluation unit 223.

The image of the fertilized ovum 16, the image capturing date and time, the image capturing condition, the second feature amount, and the growth stage (the growth stage code) are stored in association with each other, by the fertilized ovum database unit 224 (S102) .

Next, whether or not the growth stage of the fertilized ovum 16, evaluated by the evaluation unit 223 is the state of the blastocyst, in this embodiment, the state of the complete blastocyst, that is, whether or not the growth stage code is after the growth stage code 6, is determined by the determination unit 227 (S103).

In a case where it is determined as No in S103, the image capturing is ended. In a case where it is determined as YES in S103, the image of the fertilized ovum 16 is captured in a state where the fertilized ovum 16 remains still immediately before the fertilized ovum 16 is rotated, by the image capturing controller unit 2226. The shape and the position of the ICM 161 is determined according to the image recognition such as the edge detection, on the basis of the image of the fertilized ovum 16, captured immediately before the fertilized ovum 16 is rotated, by the rotation controller unit 2228. On the basis of this, the rotation direction and the rotation amount of the fertilized ovum 16 are calculated by the rotation controller unit 2228 in order to position the ICM 161 in the center portion of the fertilized ovum 16. The jet speed and the jet volume of the broth jetted from each of the jet ports, are controlled by the micro-flow path controller unit 20402, on the basis of the control signal from the rotation controller unit 2228, the rotation unit 20401 is operated, the fertilized ovum 16 is rotated (S104), and the image of the fertilized ovum 16, in which the approximately circular shape ICM 161 is positioned in the center portion, is captured by the camera 25 (S105).

Similarly, the rotation direction and the rotation amount of the fertilized ovum 16 are calculated in order to position the approximately minor arcuate ICM 161 on the end of the fertilized ovum 16. The jet speed and the jet volume of the broth jetted from each of the jet ports, are controlled by the micro-flow path controller unit 20402, on the basis of the control signal from the rotation controller unit 2228, the rotation unit 20401 is operated, the fertilized ovum 16 is rotated, and the image of the fertilized ovum 16, in which the approximately minor arcuate ICM 161 is positioned on the end, is captured by the camera 25.

Similarly, the rotation direction and the rotation amount of the fertilized ovum 16 are calculated in order to position the approximately elliptical ICM 161 on the end of the fertilized ovum 16. The jet speed and the jet volume of the broth jetted from each of the jet ports, are controlled by the micro-flow path controller unit 20402, on the basis of the control signal from the rotation controller unit 2228, the rotation unit 20401 is operated, the fertilized ovum 16 is rotated, and the image of the fertilized ovum 16 in which the approximately elliptical ICM 161 is positioned on the end, is captured by the camera 25.

Each image of the front or rear image, the lateral image, and the oblique image, captured by the camera 25, is obtained by the image obtaining unit 222. The front or rear image, the lateral image, and the oblique image of the fertilized ovum 16, the image capturing date and time, the image capturing condition, the second feature amount, and the growth stage (the growth stage code) are stored in association with each other, by the fertilized ovum database unit 224 (S106).

In a case where the N-th image is obtained when it is determined as YES in S103, the N+1-th image and the subsequence are obtained according to the flowchart illustrated in Fig. 9. In addition, in a case where it is determined as NO in S103, the next image is obtained according to the flowchart illustrated in Fig. 8, and the image is obtained according to the flowchart illustrated in Fig. 8, until it is determined as YES in S103.

Next, an obtaining method of the image subsequent to the N+1-th image will be described by using Fig. 9.

In a case where the image obtaining processing is started, the image of the fertilized ovum 16 in a state where the fertilized ovum 16 remains still immediately before the fertilized ovum 16 is rotated, is captured by the image capturing controller unit 2226. The image of the fertilized ovum 16 is obtained by the image obtaining unit 222, and the shape and the position of the ICM 161 are determined according to the image recognition such as the edge detection, by the rotation controller unit 2228, on the basis of the image of the fertilized ovum 16, captured immediately before the fertilized ovum 16 is rotated. On the basis of this, the rotation direction and the rotation amount of the fertilized ovum 16 are calculated by the rotation controller unit 2228 in order to position the ICM 161 in the center portion of the fertilized ovum 16. The jet speed and the jet volume of the broth jetted from each of the jet ports, are controlled by the rotation controller unit 2228, on the basis of the calculated rotation direction and rotation amount, the rotation unit 20401 is operated, the fertilized ovum 16 is rotated (S201), and the image of the fertilized ovum 16 in which the approximately circular shape ICM 161 is positioned in the center portion, is captured by the camera 25 (S202).

Similarly, the rotation direction and the rotation amount of the fertilized ovum 16 are calculated in order to position the approximately minor arcuate ICM 161 on the end of the fertilized ovum 16. The jet speed and the jet volume of the broth jetted from each of the jet ports are controlled by the rotation controller unit 2228, on the basis of the calculated rotation direction and rotation amount, the rotation unit 20401 is operated, the fertilized ovum 16 is rotated, and the image of the fertilized ovum 16 in which the approximately minor arcuate ICM 161 is position on the end, is captured by the camera 25.

Similarly, the rotation direction and the rotation amount of the fertilized ovum 16 are calculated in order to position the approximately elliptical ICM 161 on the end of the fertilized ovum 16. The jet speed and the jet volume of the broth jetted from each of the jet ports are calculated by the rotation controller unit 2228, on the basis of the calculated rotation direction and rotation amount, the rotation unit 20401 is operated, the fertilized ovum 16 is rotated, and the image of the fertilized ovum 16 in which the approximately elliptical ICM 161 is positioned on the end, is captured by the camera 25.

Each image of the front or rear image, the lateral image, and the oblique image, captured by the camera 25, is obtained by the image obtaining unit 222. Next, the second feature amount of the fertilized ovum 16 is extracted by the evaluation unit 223, on the basis of the image of the fertilized ovum 16, obtained by the image obtaining unit 222, and the growth stage of the fertilized ovum 16 is evaluated, and the growth stage code is applied, on the basis of the second feature amount of the fertilized ovum 16.

The front or rear image, the lateral image, and the oblique image of the fertilized ovum 16, the image capturing date and time, the image capturing condition, the second feature amount, and the growth stage (the growth stage code) are stored for each of the fertilized ova 16 in association with each other, by the fertilized ovum database unit 224 (S203).

As described above, the fertilized ovum after the growth stage of the blastocyst, having high structural asymmetry properties, is rotated, and is subjected to the image capturing a plurality of times, and thus, it is possible to obtain the images of the fertilized ovum, captured from the plurality of angles, and the evaluation accuracy is improved. In addition, in this embodiment, it is possible to control the rotation amount and the rotation direction of the fertilized ovum such that three images of the front or rear image, the oblique image, and the lateral image, in which the shapes and the positions of the ICM 161 are different from each other, can be captured, and thus, it is possible to efficiently obtain a desired image. Accordingly, it is possible to confirm a chronological change in the fertilized ovum after the growth stage of the blastocyst by arranging images at the time of capturing the image of the fertilized ovum 16 from the same angle (for example, the images of the fertilized ovum when the ICM is positioned on the front) in chronological order, and to accurately grasp the growth of the fertilized ovum, and thus, the evaluation accuracy increases.

In addition, in this embodiment, the rotation of the fertilized ovum is not performed by the manual operation, but is performed by the apparatus, and the number of times of image capturing is controlled by the information processing apparatus, according to a period of the rotation and the camera 25, and thus, it is possible to observe a large amount of fertilized ova.

### (Fourth Embodiment)

In the first embodiment and the second embodiment, the image of the rotated fertilized ovum is captured a plurality of times, and thus, the images of the fertilized ovum, captured plurality of different angles, are obtained, but a step of determining whether or not there are the images of the fertilized ovum, captured from a plurality of desired observation (image capturing) angles, may be added to the image obtaining method. Accordingly, it is possible to reliably obtain the image of the fertilized ovum, captured from a desired angle. The images of the fertilized ovum, captured from the plurality of desired observation (image capturing) angles described above, are the same images of the desired fertilized ovum 16, described in the third embodiment. In this embodiment, an observation direction sorting code is applied to three image patterns.

Note that, in the third embodiment, the same step may be added, and it is possible to evaluate whether or not the captured image is a desired image by rotating the fertilized ovum, on the basis of a calculation result of the rotation direction and the rotation amount according to the rotation controller unit. Then, in a case where there is a shift from the desired image, the result may be fed back, and the calculation result of the rotation direction and the rotation amount according to the rotation controller unit, may be corrected in order to correct the shift.

### <Observation System>

An observation system of a fourth embodiment will be described by using Fig. 4 and Fig. 5 used in the first embodiment. Fig. 4 is a diagram illustrating the configuration of the observation system. Fig. 5 is a block diagram illustrating the configuration of the observation system. In this embodiment, as with the first embodiment, a case where the rotation of the fertilized ovum 16 is performed by the manual operation, will be described as an example. Configurations different from the configurations of the first embodiment, will be mainly described, the same reference numerals will be applied to the same configurations as those of the first embodiment, and there is a case where the description thereof will be emitted.

As illustrated in Fig. 4, an observation system 3002 includes the observation apparatus 21, an information processing apparatus 3022, the display device 23, and the input device 29. In the observation apparatus 21, the light source 24, the image capturing unit 25, the temperature·humidity·gas controller unit 26, and the stage 27 are arranged.

As illustrated in Fig. 5, the information processing apparatus 3022 includes the image obtaining unit 222, an evaluation unit 3223, a fertilized ovum database unit 3224, a display controller unit 3225, the image capturing controller unit 226, and a determination unit 3227. The information processing apparatus 3022 controls the operation of each block in the observation system 3002. In this embodiment, the number of times of image capturing of the camera 25 is controlled, and the number of times of image capturing is controlled such that the image of the fertilized ovum 16 is captured from one angle up to a certain growth stage, and the image capturing is performed a plurality of times by rotating the fertilized ovum 16 to be capable of obtaining the images of the fertilized ovum 16, captured from the plurality of angles, after a certain growth stage, by the information processing apparatus 3022. In addition, fertilized ovum 16 is evaluated on the basis of the fertilized ovum image, by the information processing apparatus 3022.

The image obtaining unit 222, the evaluation unit 3223, the fertilized ovum database unit 3224, the display controller unit 3225, the image capturing controller unit 226, and the determination unit 3227 that are functional blocks of the information processing apparatus 3022, are realized by allowing the CPU to execute a program stored in the ROM that is an example of a non-transitory computer readable recording medium by loading the program in the RAM. Then, according to the functional blocks, an image obtaining method according to the present technology is executed.

The evaluation unit 3223 extracts the second feature amount of the fertilized ovum, on the basis of the image of the fertilized ovum 16, obtained by the camera 25, evaluates the growth stage of the fertilized ovum 16 by referring to the extracted second feature amount, and the first feature amount of the fertilized ovum 16 in each of the growth stages, stored in advance in the fertilized ovum database unit 3224, and applies the growth stage code described below to the fertilized ovum 16.

In addition, in the plurality of images of the fertilized ovum 16 after the growth stage code 6, as illustrated in Fig. 6(a), the evaluation unit 3223 applies a front or rear observation direction sorting code to the image in which the ICM 161 is positioned in the center portion of the fertilized ovum 16, as illustrated in Fig. 6(c), the evaluation unit 3223 applies a lateral observation direction sorting code to the image in which the approximately minor arcuate ICM 161 is positioned on the end of the fertilized ovum 16, and as illustrated in Fig. 6(b), the evaluation unit 3223 applies an oblique observation direction sorting code to the image in which the approximately elliptical ICM 161 is positioned on the end of the fertilized ovum 16.

The second feature amount of the fertilized ovum 16, calculated by the evaluation unit 3223, and the growth stage code and the observation direction sorting code, applied by the evaluation unit 3223, are associated with the images of each of the fertilized ova 16 in the fertilized ovum database unit 3224, and are stored for each of the fertilized ova 16 in chronological order.

The determination unit 3227 determines whether or not the growth stage code of the fertilized ovum 16, applied by the evaluation unit 3223, is greater than or equal to 6. In addition, the determination unit 3227 determines whether or not there is at least one of images to which each of the front or rear observation direction sorting code, the lateral observation direction sorting code, or the oblique observation direction sorting code are applied, in the plurality of captured images of the fertilized ovum 16 after the growth stage code 6.

The fertilized ovum database unit 3224 stores the image of the fertilized ovum 16, captured by the camera 25, the position information of the accommodation unit in which the fertilized ovum 16 is accommodated, for each of the accommodation units 15, along with the information such as the image capturing date and time, the image capturing condition, the second feature amount of the fertilized ovum 16, the growth stage code, the observation direction sorting code.

In addition, a plurality of first feature amounts of the image of the fertilized ovum 16 in each of the growth stages are stored in advance in the fertilized ovum database unit 3224. Further, in the fertilized ovum 16 of the growth stage after the growth stage code 6, a plurality of second feature amounts of each of the front or rear image, the lateral image, and the oblique image of the fertilized ovum 16 in each of the growth stages, are stored in advance in the fertilized ovum database unit 3224.

The display controller unit 3225 outputs a display signal for displaying the image of the fertilized ovum 16, and the image capturing date and time, the image capturing condition, the second feature amount of the fertilized ovum, the growth stage, the observation (image capturing) angle sorting, and the like, associated with the image, on the display device 23, to the display device 23.

In addition, in a case where it is evaluated that the fertilized ovum 16 is in the growth stage after the growth stage code 6, that is, the growth stage after the complete blastocyst, by the evaluation unit 3223, the display controller unit 3225 notifies the display device 23 that the fertilized ovum 16 is in the growth stage after the complete blastocyst, and displays the instruction for rotating the fertilized ovum 16 with respect to the user, on the display device 23. The user rotates the fertilized ovum 16 by the manual operation of the optical tweezers, the micropipette, or the like, or by using the water flow of the micro-flow path, the vibration, or the like, according to the instruction displayed on the display device 23. According to the start of the rotation of the fertilized ovum 16, the user inputs the start of the rotation of the fertilized ovum 16 to the information processing apparatus 3022 by using the input device 29 such as a mouse.

### <Image Obtaining Method>

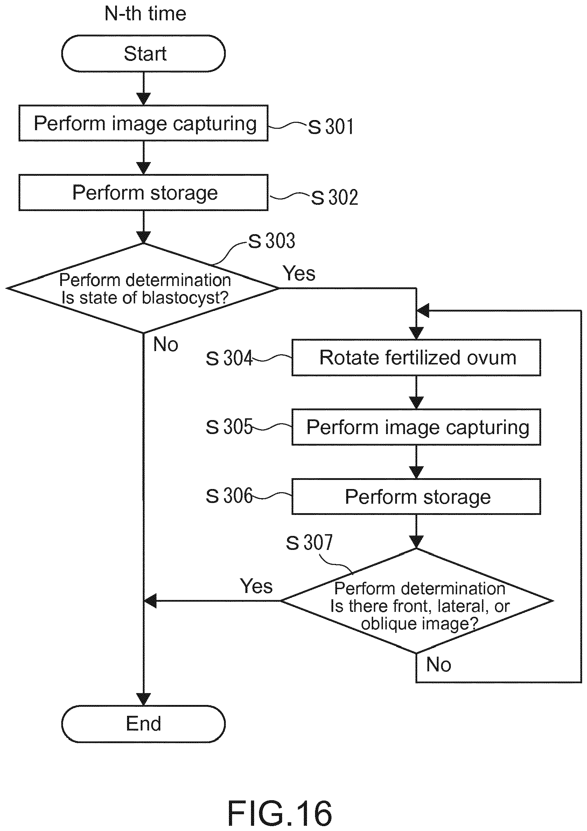

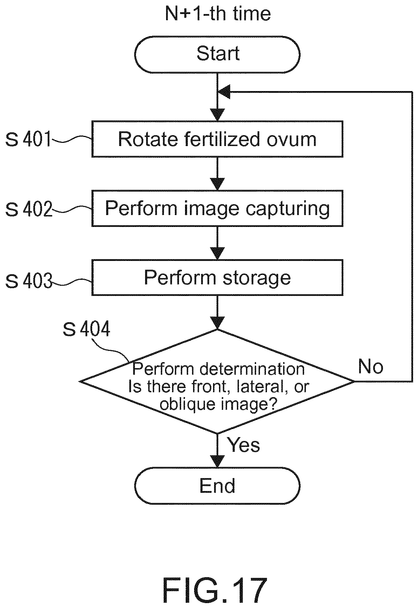

Next, an image obtaining method of the observation system 3002 described above, will be described by using Fig. 4, Fig. 16, and Fig. 17. Fig. 16 and Fig. 17 are flowcharts of the image obtaining method. Fig. 16 is an image obtaining method performed with respect to the fertilized ovum 16 up to the growth stage code 6, and Fig. 17 is an image obtaining method performed with respect to the fertilized ovum 16 after the growth stage code 7. Hereinafter, the image obtaining method will be described according to the flow of Fig. 16 and Fig. 17.

As illustrated in Fig. 4, the culture vessel 1 in which the fertilized ovum 16 of which the fertilization is confirmed, is accommodated in each of the accommodation units 15 one by one, is horizontally placed on the stage 27 in the observation apparatus 21.

Next, light from the light source 24 is emitted from the lower portion of the culture vessel 1, and the image of the fertilized ovum 16 is captured by the camera 25 positioned in the upper portion of the culture vessel 1 (S301). The camera 25 is controlled by the image capturing controller unit 226 such that the image of the fertilized ovum 16 is captured from one angle. The image captured by the camera 25, is obtained by the image obtaining unit 222.

Next, the second feature amount of the fertilized ovum 16 is extracted on the basis of the image of the fertilized ovum 16, obtained by the image obtaining unit 222, by the evaluation unit 3223. Further, the growth stage of the fertilized ovum 16 is evaluated by referring to the extracted second feature amount, and the first feature amount of the fertilized ovum 16 in each of the growth stages, stored in advance in the fertilized ovum database unit 3224, and the growth stage code is applied to the fertilized ovum 16, by the evaluation unit 223.

The image of the fertilized ovum 16, the image capturing date and time, the image capturing condition, the second feature amount, and he growth stage (the growth stage code) are stored in association with each other, by the fertilized ovum database unit 3224 (S302).

Next, whether or not the growth stage code of the fertilized ovum 16, applied by the evaluation unit 3223, is greater than or equal to 6, is determined by the determination unit 3227 (S303). In a case where it is determined as No in S303, the image capturing is ended. In a case where it is determined YES in S303, the display device 23 displays that the fertilized ovum 16 is in the growth stage of the blastocyst, and displays the instruction for rotating the fertilized ovum 16, by the display controller unit 3225 (S304).

The user confirms the instruction for rotating the fertilized ovum 16, displayed on the display device 23, and thus, rotates the fertilized ovum 16 by the manual operation of the optical tweezers, the micropipette, or the like, or by using the water flow of the micro-flow path, the vibration, or the like. The user performs the input manipulation for notifying the start of the rotation from the input device 29 immediately before the rotation of the fertilized ovum 16 is started. In a case where the input manipulation for starting the rotation is performed by the user, the camera 25 is controlled by the image capturing controller unit 226 such that the number of times of image capturing more than the number of times of image capturing at the time of capturing the images of the fertilized ovum 16 of the growth stage codes 1 to 5 so far.

The image of the fertilized ovum 16 is captured a plurality of times while the user rotates the fertilized ovum 16 (S305). The image captured by the camera 25, is obtained by the image obtaining unit 222. Next, the observation (the image capturing) direction is evaluated on the basis of the image of the fertilized ovum 16, obtained by the image obtaining unit 222, and in the plurality of obtained images, the front or rear observation direction sorting code is applied to the front or rear image the lateral observation direction sorting code is applied to the lateral image, and the oblique observation direction sorting code is applied to the oblique image, by the evaluation unit 3223.

The rotation of the fertilized ovum 16 according to the user, is ended, and the input manipulation for notifying the end of the rotation is performed from the input device 29 by the user, and thus, the image capturing of the fertilized ovum is ended. Then, each of the images of the fertilized ovum 16, the position information of the accommodation unit in which the fertilized ovum 16 is accommodated, the image capturing date and time, the image capturing condition, the second feature amount of the fertilized ovum, the growth stage (the growth stage code), the observation direction sorting code, and the like are stored in association with each other, by the fertilized ovum database unit 3224 (S306).

Next, whether or not there is at least one of the images to which each of the front or rear observation direction sorting code, the lateral observation direction sorting code, or the oblique observation direction sorting code are applied, in the plurality of images of the fertilized ovum 16 captured from the plurality of angles, is determined by the determination unit 3227 (S307). In a case where it is determined as YES in S307, the image obtaining is ended.

In a case where it is determined as NO in S307, the process returns to the step of S304, and the steps of S304, S305, S306, and S307 are repeated until it is determined as YES in S307.

In a case where the N-th image is obtained when it is determined as YES in S303, the N+1-th image and the subsequence are obtained according to the flowchart illustrated in Fig. 17. In addition, in a case where it is determined as NO in S303, the next image is obtained according to the flowchart illustrated in Fig. 16, and the image is obtained according to the flowchart illustrated in Fig. 16, until it is determined as YES in S303.

Next, an obtaining method of the image subsequent to the N+1-th image will be described by using Fig. 17.

In a case where the image processing is started, the display device 23 displays the instruction for rotating the fertilized ovum 16 by the display controller unit 3225 (S401).

The user confirms the instruction for rotating the fertilized ovum 16, displayed on the display device 23, and rotates the fertilized ovum 16 by using the optical tweezers, the micropipette, or the like. The user performs the input manipulation for notifying the start of the rotation from the input device 29 immediately before the rotation of the fertilized ovum 16 is started. In a case where the user inputs the start of the rotation, a control signal for controlling the number of times of image capturing such that one fertilized ovum 16 is captured a plurality of times, is transmitted to a number of times of image capturing controller unit 251 of the camera 25 from the image capturing controller unit 226.

The image of the fertilized ovum 16 is captured by the camera 25 a plurality of times while the user rotates the fertilized ovum 16 (S402). The image captured by the camera 25, is obtained by the image obtaining unit 222. Next, the observation (the image capturing) angle is evaluated on the basis of the image of the fertilized ovum 16, obtained by the image obtaining unit 222, and in the plurality of images, the front or rear observation direction sorting code is applied to the front or rear image, the lateral observation direction sorting code is applied to the lateral image, and the oblique observation direction sorting code is applied to the oblique image, by the evaluation unit 3223.

The rotation of the fertilized ovum 16 according to the user, is ended, and the input manipulation for notifying the end of the rotation is performed from the input device 29 by the user, and thus, the image capturing of the fertilized ovum is ended. Then, each of the images of the fertilized ovum 16, the position information of the accommodation unit in which the fertilized ovum 16 is accommodated, the image capturing date and time, the image capturing condition, the second feature amount of the fertilized ovum, the growth stage (the growth stage code), the observation direction sorting code, and the like are stored in association with each other, by the fertilized ovum database unit 3224 (S403).

Next, whether or not there is at least one of the images to which each of the front or rear observation direction sorting code, the lateral observation direction sorting code, or the oblique observation direction sorting code are applied, in the plurality of images of the fertilized ovum 16, is determined by the determination unit 3227 (S404). In a case where it is determined as YES in S404, the image obtaining is ended.

In a case where it is determined as NO in S404, the process returns to the step of S401, and the steps of S401, S402, S403, and S404 are repeated until it is determined as YES in S404.

As described above, the image capturing is performed a plurality of times by rotating the fertilized ovum after the growth stage of the blastocyst, having high structural asymmetry properties, and thus, it is possible to obtain the images of the fertilized ovum, captured from the plurality of angles. Further, in this embodiment, the image obtaining is performed until three types of images of the front or rear image, the oblique image, and the lateral image, in which the shapes and the positions of the ICM 161 are different from each other, can be image captured, and thus, it is possible to confirm a chronological change in the fertilized ovum after the growth stage of the blastocyst by arranging the images at the time of capturing the fertilized ovum from the same image capturing angle (for example, the images of the fertilized ovum when the ICM is positioned on the front) in chronological order, and to accurately grasp the growth of the fertilized ovum, and thus, the evaluation accuracy increases.

### (Fifth Embodiment)

In addition to the configurations of each of the embodiments described above, the expert (the user) such as an embryo culturer may evaluate the image of the fertilized ovum 16 in a state where the fertilized ovum 16 becomes the blastocyst, the user may determine whether or not it is the fertilized ovum that is desired to be the supervised index, and may set the fertilized ovum determined by the user that the fertilized ovum is desired to be the supervised index, as the supervised index. Then, a larger number of images may be obtained with respect to the fertilized ovum 16 that is set as the supervised index by the user, or a larger number of image information items relevant to the fertilized ovum 16 may be obtained by controlling such that a moving image is captured. In order to obtain a larger number of image information items, for example, the number of images may increase by increasing the observation (the image capturing) angle, in addition to increasing the number of images captured from the same image capturing angle.

In this embodiment, the fertilized ovum 16 determined by the user that a growth procedure of the fertilized ovum 16 is desired to be more specifically observed, of which the growth is ideal and excellent, is set as the supervised index, and the information processing apparatus learns an image signal of the fertilized ovum 16 as a supervised signal.

### <Observation System>

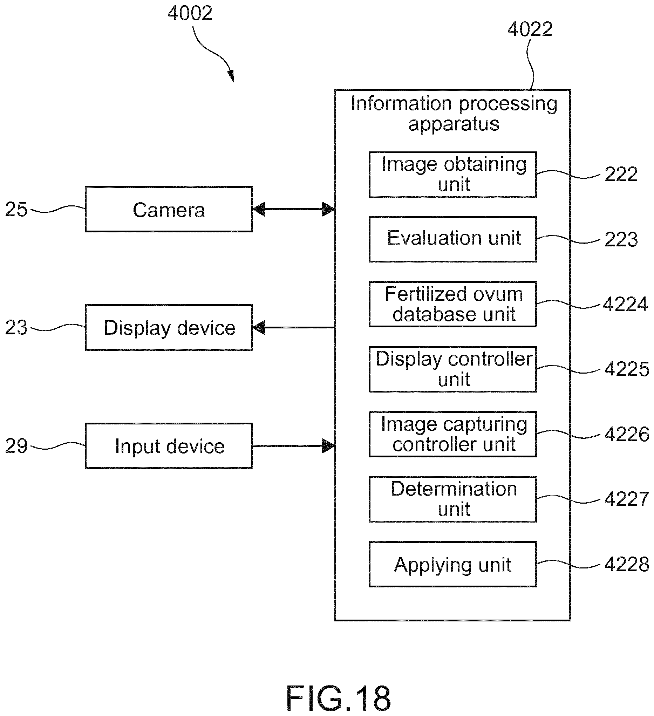

An observation system of a fifth embodiment will be described by using Fig. 4 and Fig. 18. Fig. 18 is a block diagram illustrating the configuration of the observation system. In this embodiment, as with the first embodiment, a case where the rotation of the fertilized ovum 16 is performed by the manual operation, will be described as an example. Configurations different from the configurations of the observation system of the first embodiment, will be mainly described, the same reference numerals will be applied to the same configurations, and there is a case where the description thereof will be emitted.

As illustrated in Fig. 4, an observation system 4002 includes the observation apparatus 21, an information processing apparatus 4022, the display device 23, and the input device 29. In the observation apparatus 21, the light source 24, the image capturing unit 25, the temperature·humidity·gas controller unit 26, and the stage 27 are arranged.

As illustrated in Fig. 18, the information processing apparatus 4022 includes the image obtaining unit 222, the evaluation unit 223, a fertilized ovum database unit 4224, a display controller unit 4225, an image capturing controller unit 4226, a determination unit 4227, and an applying unit 4228. The information processing apparatus 4022 controls the operation of each block in the observation system 4002. In this embodiment, the number of times of image capturing of the camera 25 is controlled, and the image capturing is controlled such that the image of the fertilized ovum 16 is captured from one angle up to the growth stage of the early blastocyst, and the image of the rotated fertilized ovum 16 is captured a plurality of times after the growth stage of the complete blastocyst, by the information processing apparatus 4022. In addition, the fertilized ovum 16 is evaluated on the basis of the image of the fertilized ovum, by the information processing apparatus 4022. In addition, an instruction for evaluating the blastocyst according to the user, is performed, by the information processing apparatus 4022.

The image obtaining unit 222, the evaluation unit 223, the fertilized ovum database unit 4224, the display controller unit 4225, the image capturing controller unit 4226, the determination unit 4227, and the applying unit 4228 that are functional blocks of the information processing apparatus 4022, are realized by allowing the CPU to execute a program stored in the ROM that is an example of a non-transitory computer readable recording medium by loading the program in the RAM. Then, according to the functional blocks, an image obtaining method according to the present technology is executed.

The determination unit 4227 determines whether or not the growth stage code of the fertilized ovum 16, applied by the evaluation unit 223, is 6. In addition, the determination unit 4227 determines whether or not the fertilized ovum 16 is applied as the supervised index by the applying unit 4228 described below. The fertilized ovum 16 applied as the supervised index, is the fertilized ovum 16 determined by the user that the fertilized ovum 16 is desired to be the supervised index.

The display controller unit 4225 outputs a display signal for displaying the image of the fertilized ovum 16, and the position information of the accommodation unit in which the fertilized ovum 16 is accommodated, the image capturing date and time, the image capturing condition, the second feature amount of the fertilized ovum, the growth stage, and the like, associated with the image, on the display device 23, to the display device 23.

In addition, in a case where the determination unit 4227 determines that the fertilized ovum 16 is in the growth stage of the growth stage code 6, that is, the growth stage of the complete blastocyst, the display controller unit 4225 displays the instruction for evaluating the blastocyst of the fertilized ovum 16 according to the expert (the user) such as an embryo culturer, on the display device 23. The user confirms the instruction displayed on the display device 23, inputs the own opinion as an evaluation value while observing the image of the fertilized ovum 16, and inputs whether or not the fertilized ovum 16 is set as the supervised index.

In addition, in a case where the determination unit 4227 determines that the fertilized ovum 16 is in the growth stage after the growth stage code 6, that is, in the growth stage after the complete blastocyst, the display controller unit 4225 controls such that the instruction for notifying that the fertilized ovum 16 is in the growth stage after the complete blastocyst, and for rotating the fertilized ovum 16 with respect to the user, is displayed on the display device 23. The user rotates the fertilized ovum 16 by using the manual operation of the optical tweezers, the micropipette, or the like, or by using the water flow according to the micro-flow path, the vibration, or the like, according to the instruction displayed on the display device 23. The user performs the input manipulation for starting the rotation of the fertilized ovum 16 by using the input device 29 such as a mouse, immediately before the rotation of the fertilized ovum 16 is started.

The applying unit 4228 applies whether or not the evaluation value of the image of the fertilized ovum 16, input by the user, is set as the supervised index, to the image of the corresponding fertilized ovum 16.

The image capturing controller unit 4226 controls the camera 25 such that the image of the fertilized ovum 16 determined that the growth stage code is after the growth stage code 6 by the determination unit 4227, is captured from one angle. The image capturing controller unit 4226 fixes the image capturing angle of the camera 25 with respect to the fertilized ovum 16 determined that the growth stage code is after the growth stage code 6, and controls the camera 25 such that the number of times of image capturing of the rotated fertilized ovum 16 is more than the number of times of image capturing of the fertilized ovum 16 of the growth stage codes 1 to 5 so far. Further, in the image capturing of the fertilized ovum 16 that is applied as the supervised index by the applying unit 4228, the image capturing controller unit 4226 controls the number of times of image capturing such that the number of times of image capturing is more than the number of times of image capturing of the other fertilized ovum 16 that is not applied as the supervised index.

The fertilized ovum database unit 4224 stores the image of the fertilized ovum 16, captured by the camera 25 for each of the accommodation units 15, along with the position information of the accommodation unit in which the fertilized ovum 16 is accommodated, the image capturing date and time, the image capturing condition, the second feature amount of the fertilized ovum 16, the growth stage, the evaluation value of the user, information of whether or not it is the supervised index, and the like. In addition, a plurality of first feature amounts of the images of the fertilized ovum 16 in each of the growth stages, are stored in advance in the fertilized ovum database unit 4224.

The fertilized ovum 16 applied as the supervised index by the applying unit 4228, for example, is the fertilized ovum 16 of which the growth is ideal and excellent, and is the fertilized ovum 16 that is desired to be the supervised index by the user. A larger number of images of the fertilized ovum 16 of which the growth is ideal and excellent, are obtained, and are set as the supervised image, and thus, it is possible to accumulate data that is the supervised index. Accordingly, it is possible to perform image evaluation with respect to the fertilized ovum 16 with a higher accuracy.

The fertilized ovum database unit 4224 stores the image of the fertilized ovum 16 that is the supervised index, arbitrarily selected by the user (the supervised image), the feature amount extracted from the image, the growth stage code, the observation angle sorting code, and the like, in association with each other, as the supervised data.

In this embodiment, the fertilized ovum 16 applied as the supervised index, arbitrarily selected by the user, is the fertilized ovum 16 of which the growth is excellent, but is not limited thereto. For example, the fertilized ovum 16 of which the growth is abnormal, may be selected as the fertilized ovum required to be subjected to more detailed image observation, as the supervised index, and the image data of the fertilized ovum 16 of which the growth is abnormal, is accumulated, and thus, for example, it is possible to find the abnormality of the early fertilized ovum. In addition, the fertilized ovum desired to be subjected to particularly detailed image observation, such as a fertilized ovum of the cattle with an excellent blood line, may be selected as the supervised index, and can be arbitrarily selected by the user.

### <Image Obtaining Method>

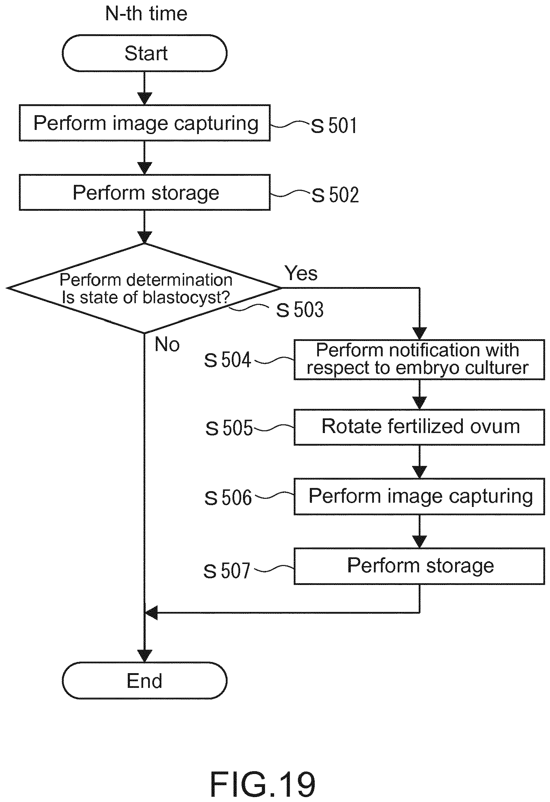

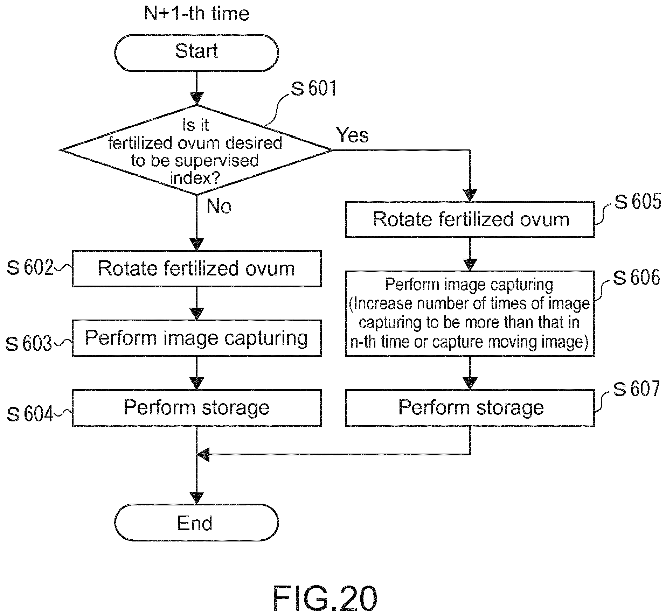

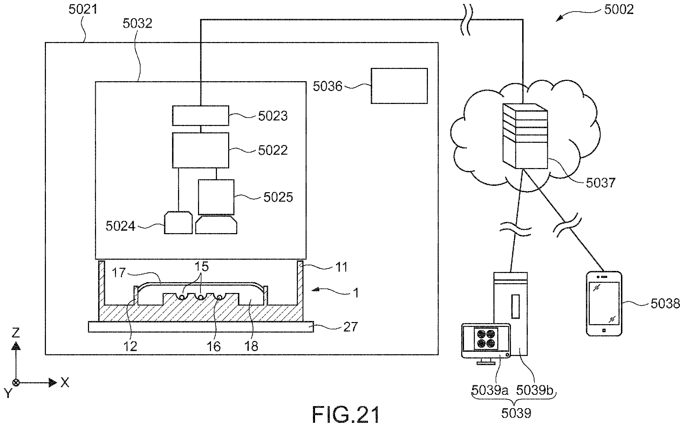

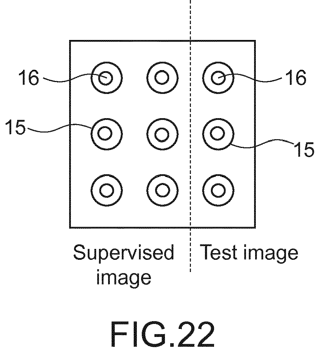

Next, an image obtaining method of the observation system 4002 described above, will be described by using Fig. 4, Fig. 19, and Fig. 20. Fig. 21 and Fig. 22 are flowcharts of the image obtaining method. Fig. 19 is an image obtaining method performed with respect to the fertilized ovum 16 up to the growth stage code 6, and Fig. 20 is an image obtaining method performed with respect to the fertilized ovum 16 after the growth stage code 7. Hereinafter, the image obtaining method will be described according to the flow of Fig. 19 and Fig. 20.

As illustrated in Fig. 4, the culture vessel 1 in which the fertilized ovum 16 of which the fertilization is confirmed, is accommodated in each of the accommodation units 15 one by one, is horizontally placed on the stage 27 in the observation apparatus 21.

Next, light from the light source 24 is emitted from the lower portion of the culture vessel 1, and the image of the fertilized ovum 16 is captured by the camera 25 positioned in the upper portion of the culture vessel 1 (S501). The camera 25 is controlled by the image capturing controller unit 4226 such that the image of the fertilized ovum 16 is captured from one angle. The image captured by the camera 25, is obtained by the image obtaining unit 222.

Next, the second feature amount of the fertilized ovum 16 is extracted on the basis of the image of the fertilized ovum 16, obtained by the image obtaining unit 222, by the evaluation unit 223. Further, the growth stage of the fertilized ovum 16 is evaluated by referring to the extracted second feature amount, and the first feature amount of the fertilized ovum 16 in each of the growth stages, stored in advance in the fertilized ovum database unit 4224, and the growth stage code is applied to the fertilized ovum 16, by the evaluation unit 223.

The image of the fertilized ovum 16, the position information of the accommodation unit in which the fertilized ovum 16 is accommodated, the image capturing date and time, the image capturing condition, the second feature amount, and the growth stage (the growth stage code) are stored in association with each other, by the fertilized ovum database unit 4224 (S502).

Next, whether or not the growth stage code of the fertilized ovum 16, applied by the evaluation unit 223, is 6, is determined by the determination unit 4227 (S503). In a case where it is determined as No in S503, the image capturing is ended. In a case where it is determined as YES in S503, the instruction for evaluating the blastocyst of the fertilized ovum 16, is displayed by the display controller unit 4225 (S504). The instruction (the notification with respect to the user) is received, and the user inputs the own opinion as the evaluation value while observing the image of the fertilized ovum 16, and inputs whether or not it is the supervised index.

Next, the display device 23 displays that the fertilized ovum 16 is in the growth stage of the early blastocyst, and displays the instruction for rotating the fertilized ovum 16, by the display controller unit 4225 (S505). The user confirms the instruction for rotating the fertilized ovum 16, displayed on the display device 23, and rotates the fertilized ovum 16 by using the manual operation of the optical tweezers, the micropipette, or the like, or by using the water flow according to the micro-flow path, the vibration, or the like.

The user performs the input manipulation for notifying the start of the rotation from the input device 29, immediately before the rotation of the fertilized ovum 16 is started. In a case where the input manipulation for starting the rotation is performed by the user, the camera 25 is controlled by the image capturing controller unit 226 such that the number of times of image capturing of the rotated fertilized ovum 16 is more than the number of times of image capturing of the fertilized ovum 16 of the growth stage codes 1 to 5 so far.

The image of the fertilized ovum 16 is captured by the camera 25 such that the number of times of image capturing is more than the number of times of image capturing at the time of capturing the image of the fertilized ovum 16 of the growth stage codes 1 to 5 so far while the user rotates the fertilized ovum 16 (S506). The image captured by the camera 25, is obtained by the image obtaining unit 222.

The rotation of the fertilized ovum 16 according to the user, is ended, and the input manipulation for notifying the end of the rotation is performed from the input device 29 by the user, and thus, the image capturing of the fertilized ovum 16 is ended. Then, the image of the fertilized ovum 16, the position information of the accommodation unit in which the fertilized ovum 16 is accommodated, the image capturing date and time, the image capturing condition, the second feature amount of the fertilized ovum, the growth stage (the growth stage code), the evaluation value of the user, the information of whether or not it is the supervised index, and the like are stored in association with each other, by the fertilized ovum database unit 4224 (S507).

In a case where the N-th image is obtained when it is determined as YES in S503, the N+1-th image and the subsequence are obtained according to the flowchart illustrated in Fig. 22. In addition, in a case where it is determined as NO in S503, the next image is obtained according to the flowchart illustrated in Fig. 19, and the image is obtained according to the flowchart illustrated in Fig. 19, until it is determined as YES in S503.

Next, an obtaining method of the image subsequent to the N+1-th image will be described by using Fig. 20.

In a case where the image processing is started, whether or not the fertilized ovum 16 that is an image capturing target, is applied as the supervised index, is determined by the determination unit 4227 (S601).

In a case where it is determined as NO in S601, the display device 23 displays the instruction for rotating the fertilized ovum 16, by the display controller unit 4225 (S602). The user confirms the instruction for rotating the fertilized ovum 16, displayed on the display device 23, and thus, rotates the fertilized ovum 16 by using the manual operation of the optical tweezers, the micropipette, or the like, or by using the water flow according to the micro-flow path, the vibration, or the like.

The user performs the input manipulation for notifying the start of the rotation from the input device 29, immediately before the rotation of the fertilized ovum 16 is started. In a case where the input manipulation for starting the rotation is performed by the user, the camera 25 is controlled by the image capturing controller unit 226 such that the number of times of image capturing of the rotated fertilized ovum 16 is more than the number of times of image capturing of the fertilized ovum 16 of the growth stage codes 1 to 5 so far.

The image of the fertilized ovum 16 is captured by the camera 25 such that the number of times of image capturing is more than the number of times of image capturing at the time of capturing the image of the fertilized ovum 16 of the growth stage codes 1 to 5 so far while the user rotates the fertilized ovum 16 (S603). The image captured by the camera 25, is obtained by the image obtaining unit 222.

The rotation of the fertilized ovum 16 according to the user, is ended, and the input manipulation for notifying the end of the rotation is performed from the input device 29 by the user, the image capturing of the fertilized ovum is ended. Then, the image of the fertilized ovum 16, the position information of the accommodation unit in which the fertilized ovum 16 is accommodated, the image capturing date and time, the image capturing condition, the second feature amount of the fertilized ovum, the growth stage (the growth stage code), the evaluation value of the user, and the information of whether or not it is the supervised index, are stored in association with each other, by the fertilized ovum database unit 4224 (S604).

In a case where it is determined as YES in S601, the display device 23 displays the instruction for rotating the fertilized ovum 16, by the display controller unit 4225 (S605). The user confirms the instruction for rotating the fertilized ovum 16, displayed on the display device 23, and thus, rotates the fertilized ovum 16 by using the manual operation of the optical tweezers, the micropipette, or the like, by using the water flow according to the micro-flow path, the vibration, or the like.

The user performs the input manipulation for notifying the start of the rotation from the input device 29, immediately before the rotation of the fertilized ovum 16 is started. In a case where the user inputs the start of the rotation, the camera 25 is controlled by the image capturing controller unit 4226 such that the number of times of image capturing of the fertilized ovum 16 is more than the number of times of image capturing in S603. Alternatively, the camera 25 may be controlled such that a moving image is captured.

The camera 25 captures the image of the fertilized ovum 16 such that the number of times of image capturing is more than the number of times of image capturing in S603 while the user rotates the fertilized ovum 16 (S606). The image captured by the camera 25, is obtained by the image obtaining unit 222.

The rotation of the fertilized ovum 16 according to the user, is ended, the input manipulation for notifying the end of the rotation is performed from the input device 29 by the user, and thus, the image capturing of the fertilized ovum is ended. Then, as the fertilized ovum 16 of the supervised index, the image or the moving image (the supervised image) of the fertilized ovum 16, the position information of the accommodation unit in which the fertilized ovum 16 is accommodated, the image capturing date and time, the image capturing condition, the feature amount of the fertilized ovum, the growth stage (the growth stage code), and the like are stored in association with each other, by the fertilized ovum database unit 4224 (S607).

In this embodiment, the user is capable of selecting the fertilized ovum of which the growth is excellent and desirable, and of evaluating the other fertilized ovum by using the selected fertilized ovum as the supervised index, and increases the number of times of image capturing of the fertilized ovum 16 that is the supervised index, to be more than the number of times of image capturing of the fertilized ovum 16 that is not the supervised index, obtains a more number of images, and obtains a large amount of supervised images from the fertilized ovum 16 that is the supervised index, and thus, it is possible to improve a determination accuracy in the machine learning.

### (Sixth Embodiment)

Next, as a sixth embodiment, an observation system having a configuration different from that of the observation systems of each of the embodiments described above, will be described. Note that, the configuration of the observation system is not limited thereto.

In the embodiment described above, the culture vessel 1 and the camera 25 are provided in the observation apparatus 21 observing the fertilized ovum, and the information processing apparatus 22 is provided outside the observation apparatus 21, but the configuration is not limited thereto. For example, as illustrated in Fig. 21, an information processing apparatus 5022 may be provided in an observation apparatus 5021.

Fig. 21 illustrates the configuration of the observation system 5002 according to the sixth embodiment. As illustrated in Fig. 21, the observation system 5002 includes an observation apparatus 5021, and the observation apparatus 5021 can be connected to a cloud server 5037 through a network. Further, a mobile terminal 5038 and a personal computer 5039 that are a display device, can be respectively connected to the cloud server 5037 through the network. In the observation apparatus 5021, a camera·information processing apparatus integrated unit 5032 and a temperature·humidity·gas controller unit 5036 are provided, and the culture vessel 1 is accommodated.

The camera·information processing apparatus integrated unit 5032 includes a camera 5025 as an image capturing unit, a light source 5024, the information processing apparatus 5022, and a communication unit 5023. In this embodiment, the light source 5024 of light to be emitted to the fertilized ovum 16, is arranged not in the lower portion but in the upper portion of the culture vessel 1.

The light source 5024 emits light to be emitted to the culture vessel 1, at the time of capturing the image of the fertilized ovum 16 in the culture vessel 1 by the camera 5025. The camera 5025 captures the image of the fertilized ovum 16 in the culture vessel 1.

The information processing apparatus described in each of the embodiments described above, can be applied to the information processing apparatus 5022. The information processing apparatus 5022 obtains the image of the fertilized ovum 16 in each of the growth stages, and for example, the image of the fertilized ovum 16 in the growth stage after the complete blastocyst, is captured in a state where the fertilized ovum 16 is rotated, and thus, the images of the fertilized ovum 16, captured from the plurality of angles, are obtained. The information processing apparatus 5022 outputs the image of the fertilized ovum 16, and the position information of the accommodation unit in which the fertilized ovum 16 is accommodated, the image capturing date and time, the image capturing condition, the second feature amount of the fertilized ovum, and a data signal such as the growth stage code and the observation direction sorting code (hereinafter, a data signal according to the fertilized ovum 16), associated with the image, to the cloud server 5037 through the communication unit 5023 and the network.

The temperature·humidity·gas controller unit 5036 controls the temperature, the humidity, and the gas in the observation apparatus 5021, and forms an environment suitable for culturing the fertilized ovum 16.

The communication unit 5023 receives the data signal according to the fertilized ovum 16, from the information processing apparatus 5022, and outputs the data signal to the cloud server 5037 through the network.

The cloud server 5037 stores the data signal according to the fertilized ovum 16. The personal computer 5039 including a display unit 5039a and an information processing unit 5039b, and the mobile terminal 5038 receives and displays the data signal according to the fertilized ovum 16 from the cloud server 5037 through the network, according to the manipulation of the user manipulating the personal computer 5039 and the mobile terminal 5038.

### (Seventh Embodiment)

In the fifth embodiment described above, the fertilized ovum selected by the user to be the supervised index, is set as the supervised index, and as with this embodiment, in the image obtaining of the fertilized ovum 16 in the growth stage after the complete blastocyst, in a plurality of fertilized ova 16 accommodated in one culture vessel 1, the image of a part of the fertilized ova 16, is set as the supervised image, and the image of the remaining fertilized ovum 16 is set as a test image, and the fertilized ovum 16 that is the supervised index, may be selected by using the images. Selection processing of the fertilized ovum 16 that is the supervised index, for example, is performed with respect to the fertilized ovum 16 in the growth stage of the complete blastocyst of the growth stage code 6.

Fig. 22 is an approximately plan view of the culture vessel 1. In this embodiment, the image of the fertilized ovum 16 accommodated in each of six accommodation units 15 in nine accommodation units 15, is set as the supervised image, and the image of the fertilized ovum 16 accommodated in each of the remaining accommodation units 15, is set as the test image.

Fig. 4 is a schematic view of the observation system according to this embodiment, and Fig. 5 is a block diagram illustrating the configuration of the observation system according to this embodiment. Hereinafter, differences from the first embodiment will be mainly described, the same reference numerals will be applied to the same structures, and there is a case where the description thereof will be emitted.

As illustrated in Fig. 4 and Fig. 5, an observation system 6002 includes the observation apparatus 21, an information processing apparatus 6022, the display device 23, and the input device 29. The light source 24, the camera 25 as an image capturing unit, the temperature·humidity·gas controller unit 26, and the stage 27 are arranged in the observation apparatus 21. The information processing apparatus 6022 includes the image obtaining unit 222, an evaluation unit 6223, a fertilized ovum database unit 6224, the display controller unit 225, the image capturing controller unit 226, and the determination unit 227.

The image obtaining unit 222, the evaluation unit 6223, the fertilized ovum database unit 6224, the display controller unit 225, the image capturing controller unit 226, and the determination unit 227 that are functional blocks of the information processing apparatus 6022, are realized by allowing the CPU to execute a program stored in the ROM that is an example of a non-transitory computer readable recording medium by loading the program in the RAM. Then, according to the functional blocks, an image obtaining method according to the present technology is executed.

The evaluation unit 6223 extracts the second feature amount, on the basis of the image of the fertilized ovum 16 of the growth stage code 6, set as the supervised image, and applies the growth stage code and the observation direction sorting code to the image of the corresponding fertilized ovum 16, on the basis of the second feature amount. In addition, the evaluation unit 6223 extracts the second feature amount, on the basis of the image of the fertilized ovum 16 of the growth stage code 6, set as the test image, and applies the growth stage code and the observation direction sorting code to the image of the corresponding fertilized ovum 16, on the basis of the second feature amount.

The evaluation unit 6223 evaluates the second feature amount of the fertilized ovum 16, set as the supervised image, and reliability and usefulness of a relationship between the growth stage code and the observation direction sorting code, by using a set of the second feature amount of the fertilized ovum 16, set as the test image, and the growth stage code and the observation direction sorting code. In a plurality of fertilized ova 16 set as the supervised image, the fertilized ovum 16 evaluated as being useful, is set as the supervised index by the evaluation unit 6223, and the image (the supervised image), the feature amount, the growth stage code and the observation direction sorting code are stored in association with each other, as the supervised data, by the fertilized ovum database unit 6224. In the subsequent image obtaining, the feature amount is used as the first feature amount, and the growth stage or the image capturing angle is evaluated by referring to the supervised data obtained at the time of obtaining the image.

### (Eighth Embodiment)

In the embodiment described above, the captured image of the growth stage after the complete blastocyst, is sorted into three image patterns by using the observation (the image capturing) direction as a sorting index, and as with this embodiment, the sorting may be performed by using the observation (the image capturing) angle in which further the observation (the image capturing) direction is more specifically sorted, as the sorting index. In an eighth embodiment, the observation angle sorting code is provided, the images of the fertilized ovum, captured from the plurality of angles after the growth stage code 6, are sorted by using the observation (the image capturing) angle as the sorting index, and the observation angle sorting code is applied to the obtained image. The application of the observation angle sorting code can be applied to each of the embodiments described above. In this embodiment, as with the first embodiment, a case where the rotation of the fertilized ovum 16 is performed by the manual operation, will be described as an example. Configurations different from those of the observation system of the first embodiment, will be mainly described, the same reference numerals will be applied to the same configurations, and there is a case where the description thereof will be emitted.

### <Observation Angle Sorting>

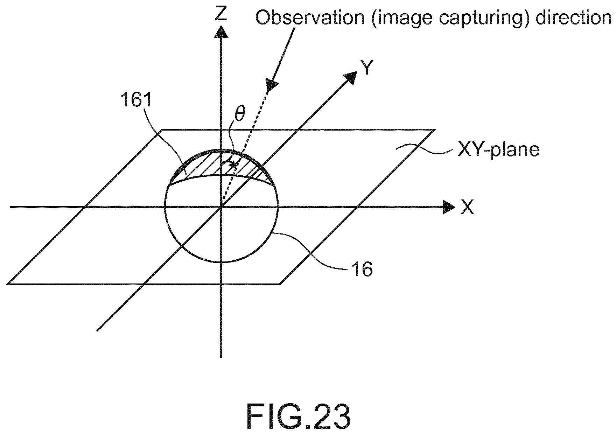

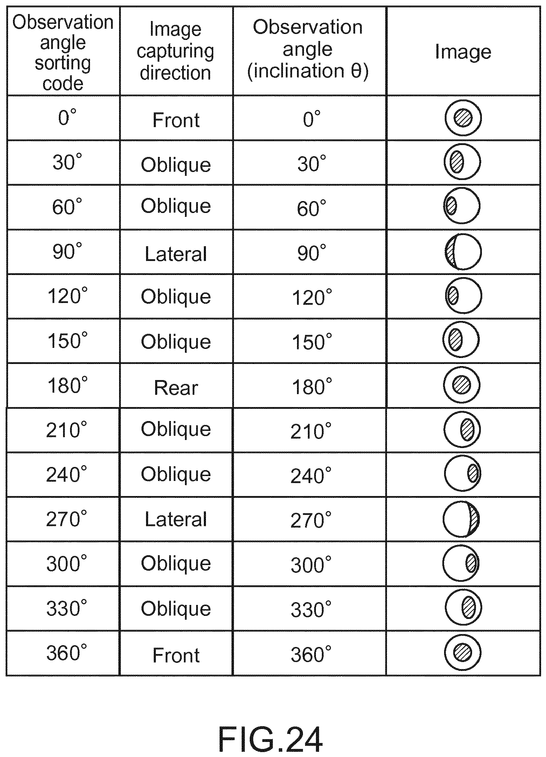

Fig. 23 and Fig. 24 are diagrams for describing the sorting according to the observation angle. Fig. 23 is a diagram illustrating the definition of the observation (the image capturing) angle, and Fig. 24 is a diagram illustrating a correspondence in the observation angle sorting code, the observation (the image capturing) direction, the observation (the image capturing) angle, and the image of the fertilized ovum. In this embodiment, the captured image is sorted according to the observation (the image capturing) angle of the fertilized ovum 16, and the observation angle sorting code is applied to each of the images.

As illustrated in Fig. 23, centering around an approximately spherical fertilized ovum 16, on the basis of the observation (the image capturing) direction (a direction from the plus towards the minus of the Z-axis, in Fig. 23) at the time of capturing the image of the fertilized ovum 16 from the front, an angle θ between the observation direction from the front, and the observation (the image capturing) direction, is set to the observation (the image capturing) angle, and the observation angle sorting code is applied corresponding to the observation (the image capturing) angle. The observation angle sorting code is represented by a numerical value of 0° to 360°. Note that, in Fig. 23, the X-axis, the Y-axis, and the Z-axis indicate three axes orthogonal to each other, and do not indicate the horizontal direction and the vertical direction.

As described above, in the growth stage of the fertilized ovum 16 after the blastocyst, the ICM 161 biasedly exists in the fertilized ovum 16.

As illustrated in Fig. 23, in a state where the fertilized ovum 16 is arranged such that in XYZ coordinates, the center of the approximately spherical fertilized ovum 16 is positioned at the origin point, the ICM 161 is positioned in the plus region of the Z-axis, and the center of the ICM 161 of which the planar shape is an approximately circular shape at the time of projecting the ICM 161 onto the XY-plane, is positioned at the origin point, the image of the fertilized ovum 16 at the time of being captured in the observation (the image capturing) direction from the plus towards the minus of the Z-axis along the Z-axis, as illustrated in Fig. 24, corresponds to the front image of the fertilized ovum in which the ICM 161 is positioned on the front, and the observation angle sorting code of 0° is applied to the image.

In Fig. 23, the image of the fertilized ovum 16, captured in the direction from the minus towards the plus of the Z-axis along the Z-axis, in other words, the image of the fertilized ovum 16, captured from the rear side, as illustrated in Fig. 24, is an image in which the ICM 161 of which the planar shape is the approximately circular shape, is positioned in the center portion of the fertilized ovum 16, and the observation angle sorting code of 180° is applied to the rear image. Here, both of the front image and the rear image are the image in which an approximately circular shape ICM 161 is positioned in the center portion of the fertilized ovum 16, but the front image and the rear image, for example, can be discriminated from each other by a contrasting density.

In Fig. 23, the image of the fertilized ovum 16, captured in the observation (the image capturing) direction from the position in the XY-plane towards the origin point, in other words, the image of the fertilized ovum 16, captured from the lateral side, as illustrated in Fig. 24, is an image in which an approximately minor arcuate ICM 161 is positioned on the end of the fertilized ovum 16 of which the planar shape is an approximately circular shape, and the observation angle sorting code of 90° is applied to the image.

In Fig. 23, the image of the fertilized ovum 16, captured in the observation (the image capturing) direction where the observation (the image capturing) angle θ is 0° < θ < 90°, 90° < θ < 180°, 180° < θ < 270°, and 270° < θ < 360°, in other words, the image of the fertilized ovum 16, captured from the oblique side in addition to the front side, the rear side, and the lateral side, is an image in which an approximately elliptical ICM 161 is positioned in a position shifted from the center of the fertilized ovum 16, and the size or the shape of the ICM 161 is different according to the observation (the image capturing) angle. The observation angle sorting code according to the observation (the image capturing) angle, is applied to the image.

As illustrated in Fig. 24, for example, the image of the fertilized ovum 16, having the observation angle sorting code of 30°, captured at the observation (the image capturing) angle of 30°, is an image in which the approximately elliptical ICM 161 close to a circular shape, is positioned in a position slightly shifted from the center of the fertilized ovum 16. The image captured at the observation (the image capturing) angle of 30°, is an image in which the position of the ICM 161, for example, is different such as the upper right side or the lower side of the fertilized ovum 16, according to the observation (the image capturing) direction in the XY-plane at the time of projecting the observation (the image capturing) direction into the XY-plane. However, in a case where the observation (the image capturing) angle is the same, the shape or the size of the ICM 161 is approximately the same, and in a case where images captured from different directions at the same observation (the image capturing) angle are rotated and moved around the center of an approximately circular fertilized ovum 16, the ICMs 161 are approximately coincident with each other. The same applies to other observation (image capturing) angles.

As illustrated in Fig. 24, for example, the image of the fertilized ovum 16, having the observation angle sorting code of 60°, captured at the observation (the image capturing) angle of 60°, is an image in which the approximately elliptical ICM 161 is positioned on the end of the fertilized ovum 16. In addition, for example, the image of the fertilized ovum 16, having the observation angle sorting code of 120°, captured at the observation (the image capturing) angle of 120°, is an image in which the approximately elliptical ICM 161 is positioned on the end of the fertilized ovum 16. In addition, for example, the image of the fertilized ovum 16, having the observation angle sorting code of 150°, captured at the observation (the image capturing) angle of 150°, is an image in which the approximately elliptical ICM 161 close to the circular shape, is positioned in a position slightly shifted from the center of the fertilized ovum 16.

In addition, in an image captured at an oblique observation (image capturing) angle, the observation (the image capturing) angle can be calculated from the size (the area) or the shape of the ICM 161, the position of the ICM 161, seen from the center of the fertilized ovum 16 of which the planar shape is the approximately circular shape, and the like.

Here, for example, both of the image having the observation angle sorting code of 30° and the image having the observation angle sorting code of 150°, are the image in which the approximately elliptical ICM 161 close to the circular shape, is positioned in the position slightly shifted from the center of the fertilized ovum 16, but both of the images, for example, can be discriminated by the contrasting density.

In addition, both of the image having the observation angle sorting code of 60° and the image having the observation angle sorting code of 120°, are the image in which the approximately elliptical ICM 161 is positioned on the end of the fertilized ovum 16, but both of the images, for example, can be discriminated from each other by the contrasting density.

As described above, in this embodiment, the images obtained according to the image capturing of the rotated fertilized ovum 16, are sorted according to the observation (the image capturing) angle, and thus, the images to be observed (captured) from the oblique direction, can be more specifically sorted. Accordingly, it is possible to obtain more detailed image information of the fertilized ovum 16, and to perform the evaluation of the fertilized ovum 16 with a high accuracy. Note that, in the above description, the observation (the image capturing) angle has been described at an interval of 30°, but is not limited thereto.

### <Observation System>

An observation system of the eighth embodiment will be described by using Fig. 4 and Fig. 5 used in the first embodiment. Fig. 4 is a diagram illustrating the configuration of the observation system. Fig. 5 is a block diagram illustrating the configuration of the observation system. In this embodiment, as with the first embodiment, a case where the rotation of the fertilized ovum 16 is performed by the manual operation, will be described as an example. Configurations different from those of the observation system of the first embodiment, will be mainly described, the same reference numerals will be applied to the same configurations, and there is a case where the description thereof will be emitted.

As illustrated in Fig. 4, an observation system 7002 includes the observation apparatus 21, an information processing apparatus 7022, the display device 23, and the input device 29. The light source 24, the image capturing unit 25, the temperature·humidity·gas controller unit 26, and the stage 27 are arranged in the observation apparatus 21.

As illustrated in Fig. 5, the information processing apparatus 7022 includes the image obtaining unit 222, an evaluation unit 7223, a fertilized ovum database unit 7224, the display controller unit 225, the image capturing controller unit 226, and the determination unit 3227. The information processing apparatus 7022 controls the operation of each block in the observation system 7002. In this embodiment, the number of times of image capturing of the camera 25 is controlled, and the image capturing is controlled such that the image of the fertilized ovum 16 is captured from one angle up to a certain growth stage, and the image of the rotated fertilized ovum 16 is captured a plurality of times after a certain growth stage, by the information processing apparatus 7022. In addition, the fertilized ovum 16 is evaluated on the basis of the image of the fertilized ovum, by the information processing apparatus 7022.

The image obtaining unit 222, the evaluation unit 7223, the fertilized ovum database unit 7224, the display controller unit 225, the image capturing controller unit 226, and the determination unit 227 that are functional blocks of the information processing apparatus 7022, are realized by allowing the CPU to execute a program stored in the ROM that is an example of a non-transitory computer readable recording medium by loading the program in the RAM. Then, according to the functional blocks, an image obtaining method according to the present technology is executed.

The evaluation unit 7223 extracts the second feature amount of the fertilized ovum, on the basis of the image of the fertilized ovum 16, obtained by the camera 25, evaluates the growth stage of the fertilized ovum 16 by referring to the extracted second feature amount, and the first feature amount of the fertilized ovum 16 in each of the growth stages, stored in advance in the fertilized ovum database unit 7224, and applies the growth stage code described below, to the fertilized ovum 16.

In addition, the evaluation unit 7223 extracts the second feature amount of the fertilized ovum, on the basis of the image, for each of the images of the fertilized ovum 16, captured from the plurality of image capturing angles after the growth stage code 6, evaluates the observation (the image capturing) angle by referring to the extracted second feature amount, and the first feature amount of the fertilized ovum 16 in each of the growth stages, stored in advance in the fertilized ovum database unit 7224, and applies the observation angle sorting code.

The second feature amount of the fertilized ovum 16, calculated by the evaluation unit 7223, and the growth stage code and the observation angle sorting code, applied by the evaluation unit 7223, are associated with the images of each of the fertilized ova 16 in the fertilized ovum database unit 7224, and are stored for each of the fertilized ova 16 in chronological order.

The determination unit 227 determines whether or not the growth stage code of the fertilized ovum 16, applied by the evaluation unit 7223, is after the growth stage code 6.

The fertilized ovum database unit 7224 stores the image of the fertilized ovum 16, captured by the camera 25, for each of the accommodation units 15, along with the information such as position information of the accommodation unit in which the fertilized ovum 16 is accommodated, the image capturing date and time, the image capturing condition, the second feature amount of the fertilized ovum 16, the growth stage code, and the observation angle sorting code.

In addition, a plurality of first feature amounts of the image of the fertilized ovum 16 in each of the growth stages, are stored in advance in the fertilized ovum database unit 7224. Further, in the fertilized ovum 16 in the growth stage after the growth stage code 6, a plurality of second feature amounts of the image of the fertilized ovum 16 in each of the growth stages at each of the observation (the image capturing) angles, are stored in advance in the fertilized ovum database unit 7224.

The display controller unit 225 outputs a display signal for displaying the image of the fertilized ovum 16, and the image capturing date and time, the image capturing condition, the second feature amount of the fertilized ovum, the growth stage, the observation (the image capturing) angle, and the like, associated with the image, on the display device 23, to the display device 23.

In addition, in a case where the evaluation unit 7223 evaluates that the fertilized ovum 16 is in the growth stage after the growth stage code 6, that is, the growth stage after the complete blastocyst, the display controller unit 225 displays the instruction for notifying that the fertilized ovum 16 is in the growth stage after the complete blastocyst, and for rotating the fertilized ovum 16 with respect to the user, on the display device 23. The user rotates the fertilized ovum 16 by using the optical tweezers, the micropipette, or the like, according to the instruction displayed on the display device 23. The user inputs the start of the rotation of the fertilized ovum 16 into the information processing apparatus 7022 by using the input device 29 such as a mouse, according to the start of the rotation of the fertilized ovum 16.

### <Image Obtaining Method>

Next, an image obtaining method of the observation system 7002 described above, will be described, but is different from the image obtaining method of the first embodiment, in that the images of the fertilized ovum, captured in a state of being rotated, are sorted at each of the observation (the image capturing) angles, and the observation angle sorting code is applied, and will be described below by using Fig. 8 and Fig. 9 that are the same flowchart as that of the first embodiment.

As illustrated in Fig. 4, the culture vessel 1 in which the fertilized ovum 16 of which the fertilization is confirmed, is accommodated in each of the accommodation units 15 one by one, is horizontally placed on the stage 27 in the observation apparatus 21.

Next, light from the light source 24 is emitted from the lower portion of the culture vessel 1, and the image of the fertilized ovum 16 is captured by the camera 25 positioned in the upper portion of the culture vessel 1 (S101). The camera 25 is controlled by the image capturing controller unit 226 such that the images of the fertilized ovum 16 is captured from one angle. The image captured by the camera 25, is obtained by the image obtaining unit 222.

Next, the second feature amount of the fertilized ovum 16 is extracted on the basis of the image of the fertilized ovum 16, obtained by the image obtaining unit 222, by the evaluation unit 7223. Further, the growth stage of the fertilized ovum 16 is evaluated by referring to the extracted second feature amount, and the first feature amount of the fertilized ovum 16 in each of the growth stages, stored in advance in the fertilized ovum database unit 7224, and the growth stage code is applied to the fertilized ovum 16, by the evaluation unit 7223.

The image of the fertilized ovum 16, the image capturing date and time, the image capturing condition, the second feature amount, and the growth stage (the growth stage code) are stored in association with each other, by the fertilized ovum database unit 7224 (S102).

Next, whether or not the growth stage code of the fertilized ovum 16, applied by the evaluation unit 7223, is after the growth stage code 6, is determined by the determination unit 227 (S103). In a case where it is determined as No in S103, the image capturing is ended. In a case where it is determined as YES in S103, the display device 23 displays that the fertilized ovum 16 is in the growth stage of the blastocyst, and displays the instruction for rotating the fertilized ovum 16, by the display controller unit 225 (S104).

The user confirms the instruction for rotating the fertilized ovum 16, displayed on the display device 23, and thus, rotates the fertilized ovum 16 by using the manual operation of the optical tweezers, the micropipette, or the like, and by using the water flow according to the micro-flow path, the vibration, or the like.

The user performs the input manipulation for notifying the start of the rotation from the input device 29 immediately before the rotation of the fertilized ovum 16 is started. In a case where the input manipulation for starting the rotation is performed by the user, and the camera 25 is controlled by the image capturing controller unit 226 such that the number of times of image capturing of the rotated fertilized ovum 16 is more than the number of times of image capturing of the fertilized ovum 16 of the growth stage codes 1 to 5 so far. Hereinafter, the number of times of image capturing of the fertilized ovum 16 after the growth stage code 6, is more than the number of times of image capturing up to the growth stage code 5.

The images of the fertilized ovum 16 are captured by the camera 25 a plurality of times while the user rotates the fertilized ovum 16 (S105). The image captured by the camera 25, is obtained by the image obtaining unit 222. Next, the second feature amount of the fertilized ovum 16 is extracted on the basis of the image of the fertilized ovum 16, obtained by the image obtaining unit 222, by the evaluation unit 7223. Further, the observation (the image capturing) angle is evaluated by referring to the extracted second feature amount, and the first feature amount of the fertilized ovum 16 in each of the growth stages, stored in advance in the fertilized ovum database unit 3224, and the observation angle sorting code is applied, by the evaluation unit 7223.

The rotation of the fertilized ovum 16 according to the user, is ended, and the input manipulation for notifying the end of the rotation is performed from the input device 29 by the user, and thus, the image capturing of the fertilized ovum is ended. Then, each of the images of the fertilized ovum 16, the position information of the accommodation unit in which the fertilized ovum 16 is accommodated, the image capturing date and time, the image capturing condition, the second feature amount of the fertilized ovum, the growth stage (the growth stage code), the observation angle sorting code, and the like are stored in association with each other, by the fertilized ovum database unit 7224 (S106).

In a case where the N-th image is obtained when it is determined as YES in S103, the N+1-th image and the subsequence are obtained according to the flowchart illustrated in Fig. 9. In addition, in a case where it is determined as NO in S103, the next image is obtained according to the flowchart illustrated in Fig. 8, and the image is obtained according to the flowchart illustrated in Fig. 8, until it is determined as YES in S103.

Next, an obtaining method of the image subsequent to the N+1-th image will be described by using Fig. 9.

In a case where the image processing is started, the instruction for rotating the fertilized ovum 16 is displayed on the display device 23, by the display controller unit 225 (S201). The user confirms the instruction for rotating the fertilized ovum 16, displayed on the display device 23, and rotates the fertilized ovum 16 by using the manual operation of the optical tweezers, the micropipette, or the like, or by using the water flow according to the micro-flow path, the vibration, or the like.

The user performs the input manipulation for notifying the start of the rotation from the input device 29, immediately before the rotation of the fertilized ovum 16 is started. In a case where the user inputs the start of the rotation, the camera 25 is controlled by the image capturing controller unit 226 such that the number of times of image capturing of the rotated fertilized ovum 16 is more than the number of times of image capturing of the fertilized ovum 16 of the growth stage codes 1 to 5 so far.

The image of the fertilized ovum 16 is captured a plurality of times while the user rotates the fertilized ovum 16 (S202). The image captured by the camera 25, is obtained by the image obtaining unit 222. Next, the second feature amount of the fertilized ovum 16 is extracted on the basis of the image of the fertilized ovum 16, obtained by the image obtaining unit 222, and the observation (the image capturing) angle is evaluated, by the evaluation unit 7223. Further, the observation angle sorting code is applied to each of the images by the evaluation unit 7223.

The rotation of the fertilized ovum 16 is ended by the user, and the input manipulation for notifying the end of the rotation is performed from the input device 29 by the user, and thus, the image capturing of the fertilized ovum is ended. Then, each of the images of the fertilized ovum 16, the position information of the accommodation unit in which the fertilized ovum 16 is accommodated, the image capturing date and time, the image capturing condition, the second feature amount of the fertilized ovum 16, the growth stage (the growth stage code), the observation angle sorting code, and the like are stored in association with each other by the fertilized ovum database unit 7224 (S203).

As described above, the captured images are sorted according to the observation (the image capturing) angle, and thus, it is possible to obtain more detailed image data. In addition, this embodiment can be applied to each of the embodiments described above.

In addition, in this embodiment, the observation angle sorting code is applied to the image of the fertilized ovum 16 in the growth stage after the growth stage code 6, but the observation angle sorting code may be applied to the image of the fertilized ovum 16 in the growth stage before the growth stage code 5. Then, in the image of the fertilized ovum 16 that is the supervised index, before the growth stage code 5, an image to which a specific observation angle sorting code is applied, may be used as the supervised image. At this time, in the fertilized ovum 16 in the growth stage up to the growth stage code 5, it is desirable that the observation angle sorting code is defined in each of the growth stages, according to the feature of the shape of the fertilized ovum 16 in each of the growth stages.

For example, the 2-cell period will be exemplified. The observation angle sorting code of 0° is applied to a front image in which two cells are adjacent to each other without overlapping each other, as illustrated in Fig. 3(b), the observation angle sorting code of 90°, corresponding to the lateral image, is applied to one elliptical image, the oblique observation angle sorting code of 0° < θ < 90°, 90° < θ < 180°, 180° < θ < 270°, and 270° < θ < 360° is applied to an image in which two cells partially overlap each other. Then, in the image of the 2-cell period of the fertilized ovum 16 that is the supervised index, an image to which one specific observation angle sorting code, for example, the observation angle sorting code of 0° is applied, is set as the supervised image.

Thus, in the fertilized ovum 16 that is the supervised index, before the growth stage code 5, an image to which one specific observation angle sorting code is applied, may be set as the supervised image, and in the fertilized ovum after the growth stage code 6, a plurality of images to which a plurality of observation angle sorting codes are respectively applied, may be set as the supervised image.

As described above, each of the embodiments of the present technology has been described, but the present technology is not limited to the embodiments described above, and various changes can be added to the present technology within a range not departing from the gist of the present technology.

In the embodiments described above, in the growth stage after the complete blastocyst, the images of the fertilized ovum are captured from the plurality of angles, but the configuration is not limited thereto.

In addition, in the embodiments described above, the evaluation unit evaluates the growth stage on the basis of the image of the fertilized ovum, and applies the growth stage code to the fertilized ovum, and the determination unit determines whether or not the growth stage code is after the growth stage code 6, and determines whether or not the fertilized ovum is rotated, but the evaluation of the growth stage is not limited thereto.

For example, the evaluation unit may evaluate the growth stage on the basis of a culture time of the fertilized ovum, that is, the elapsed time from a fertilization date, and may apply the growth stage code to the fertilized ovum, and the determination unit may determine whether or not the growth stage code is after the growth stage code 6, and may determine whether or not the fertilized ovum is rotated. The general fertilized ovum grows through the growth stage as illustrated in Fig. 3, and it is almost clear in which growth stage the fertilized ovum is at the elapsed time from the fertilization date (the culture time). Therefore, it is possible to evaluate the growth stage from the culture time of the fertilized ovum.

Note that, the present technology is capable of having the following configurations.
(1) An information processing apparatus, including:
   an image obtaining unit that obtains an image of a cell, the image being captured by an image capturing unit;
   an evaluation unit that evaluates a growth stage of the cell; and
   an image capturing controller unit that allows the image capturing unit to capture an image of the rotated cell, according to an evaluation result of the evaluation unit.
(2) The information processing apparatus according to (1) described above, further including
   a rotation controller unit that controls a rotation mechanism that rotates the cell, according to the evaluation result of the evaluation unit.
(3) The information processing apparatus according to (1) or (2) described above, in which
   the evaluation unit evaluates the growth stage of the cell on a basis of the image obtained by the image obtaining unit.
(4) The information processing apparatus according to (1) or (2) described above, in which
   the evaluation unit evaluates the growth stage of the cell on a basis of a culture time of the cell.
(5) The information processing apparatus according to any one of (1) to (4) described above, in which
   when the evaluation unit evaluates that the cell is in a growth stage in which the cell has an asymmetric shape, the image capturing controller unit allows the image capturing unit to capture the image of the rotated cell.
(6) The information processing apparatus according to (5) described above, in which
   the growth stage in which the cell has the asymmetric shape is a growth stage of a blastocyst.
(7) The information processing apparatus according to (6) described above, in which
   the growth stage of the blastocyst is a growth stage after a complete blastocyst.
(8) The information processing apparatus according to any one of (1) to (7) described above, in which
   the cell is a cell in the growth stage of the blastocyst having an inner cell mass, and
   the evaluation unit sorts the captured image of the rotated cell, according to an image capturing direction based on a position of the inner cell mass in the cell.
(9) The information processing apparatus according to any one of (1) to (8) described above, further including
   a storage unit that stores the image of the cell, the image being obtained by the image obtaining unit, in which
   the storage unit stores in advance a first feature amount of the cell in each growth stage, and
   the evaluation unit extracts a second feature amount of the image of the cell, the image being obtained by the image obtaining unit, and evaluates the growth stage of the cell on a basis of the first feature amount and the second feature amount.
(10) The information processing apparatus according to (9) described above, in which
   the storage unit stores the arbitrarily selected cell as a supervised index.
(11) The information processing apparatus according to (10) described above, in which
   the image capturing controller unit controls the image capturing unit such that the image capturing unit captures images of the plurality of cells, and
   the image capturing controller unit controls the image capturing unit such that the number of times of image capturing of the cell that is set as the supervised index is more than the number of times of image capturing of the cell that is not set as the supervised index.
(12) The information processing apparatus according to any one of (1) to (9) described above, in which
   the image capturing controller unit controls the image capturing unit such that the image capturing unit captures images of the plurality of cells, and
   the evaluation unit sets images of a part of cells in the plurality of cells, as a supervised image, and images of other cells in the plurality of cells, as a test image, and verifies the supervised image by using the test image.
(13) An observation system, including:
   a culture vessel including a plurality of accommodation units in which a cell is accommodated;
   an image capturing unit that captures an image of the cell;
   an image obtaining unit that obtains the image of the cell, the image being captured by the image capturing unit;
   an evaluation unit that evaluates a growth stage of the cell;
   a rotation mechanism that rotates the cell in the accommodation unit, according to an evaluation result of the growth stage of the cell of the evaluation unit; and
   an image capturing controller unit that controls the image capturing unit such that the image capturing unit captures an image of the cell rotated by the rotation mechanism.
(14) The observation system according (13) described above, in which
   the rotation mechanism is a vibration apparatus that vibrates the culture vessel.
(15) The observation system according (13) described above, in which
   the accommodation unit is capable of accommodating the cell and a liquid,
   the culture vessel includes the rotation mechanism, and
   the rotation mechanism rotates the cell by generating a flow in the liquid in the accommodation unit.
(16) An information processing method, including:
   evaluating a growth stage of a cell; and
   capturing an image of the rotated cell when it is evaluated that the cell is in a growth stage in which the cell has an asymmetric shape.
(17) A program causing an information processing apparatus to execute:
   a step of evaluating a growth stage of a cell; and
   a step of capturing an image of the rotated cell when it is evaluated that the cell is in a growth stage in which the cell has an asymmetric shape.

### Reference Signs List

1, 2040 culture vessel
2, 1002, 2002, 3002, 4002, 5002, 6002, 7002 observation system
15, 2015 accommodation unit
16 fertilized ovum
22, 1022, 2022, 3022, 4022, 5022, 6022, 7022 information processing apparatus
25, 5025 camera (image capturing unit)
161 inner cell mass (ICM)
222 image obtaining unit
223, 3223, 6223, 7223 evaluation unit
224, 3224, 4224, 6224, 7224 fertilized ovum database unit (storage unit)
226, 2226, 4226 image capturing controller unit
1040 vibration apparatus (rotation mechanism)
1608 early blastocyst
1609 complete blastocyst
1610 expanded blastocyst
1611 hatched blastocyst
1612 expanded hatched blastocyst
20401 rotation unit (rotation mechanism)

## Claims

1. An information processing apparatus, comprising:
an image obtaining unit that obtains an image of a cell, the image being captured by an image capturing unit;
an evaluation unit that evaluates a growth stage of the cell; and
an image capturing controller unit that allows the image capturing unit to capture an image of the rotated cell, according to an evaluation result of the evaluation unit.

2. The information processing apparatus according to claim 1, further comprising
a rotation controller unit that controls a rotation mechanism that rotates the cell, according to the evaluation result of the evaluation unit.

3. The information processing apparatus according to claim 2, wherein
the evaluation unit evaluates the growth stage of the cell on a basis of the image obtained by the image obtaining unit.

4. The information processing apparatus according to claim 2, wherein
the evaluation unit evaluates the growth stage of the cell on a basis of a culture time of the cell.

5. The information processing apparatus according to claim 3, wherein
when the evaluation unit evaluates that the cell is in a growth stage in which the cell has an asymmetric shape, the image capturing controller unit allows the image capturing unit to capture the image of the rotated cell.

6. The information processing apparatus according to claim 5, wherein
the growth stage in which the cell has the asymmetric shape is a growth stage of a blastocyst.

7. The information processing apparatus according to claim 6, wherein
the growth stage of the blastocyst is a growth stage after a complete blastocyst.

8. The information processing apparatus according to claim 7, wherein
the cell is a cell in the growth stage of the blastocyst having an inner cell mass, and
the evaluation unit sorts the captured image of the rotated cell, according to an image capturing direction based on a position of the inner cell mass in the cell.

9. The information processing apparatus according to claim 8, further comprising
a storage unit that stores the image of the cell, the image being obtained by the image obtaining unit, wherein
the storage unit stores in advance a first feature amount of the cell in each growth stage, and
the evaluation unit extracts a second feature amount of the image of the cell, the image being obtained by the image obtaining unit, and evaluates the growth stage of the cell on a basis of the first feature amount and the second feature amount.

10. The information processing apparatus according to claim 9, wherein
the storage unit stores the arbitrarily selected cell as a supervised index.

11. The information processing apparatus according to claim 10, wherein
the image capturing controller unit controls the image capturing unit such that the image capturing unit captures images of the plurality of cells, and
the image capturing controller unit controls the image capturing unit such that the number of times of image capturing of the cell that is set as the supervised index is more than the number of times of image capturing of the cell that is not set as the supervised index.

12. The information processing apparatus according to claim 9, wherein
the image capturing controller unit controls the image capturing unit such that the image capturing unit captures images of the plurality of cells, and
the evaluation unit sets images of a part of cells in the plurality of cells, as a supervised image, and images of other cells in the plurality of cells, as a test image, and verifies the supervised image by using the test image.

13. An observation system, comprising:
a culture vessel including a plurality of accommodation units in which a cell is accommodated;
an image capturing unit that captures an image of the cell;
an image obtaining unit that obtains the image of the cell, the image being captured by the image capturing unit;
an evaluation unit that evaluates a growth stage of the cell;
a rotation mechanism that rotates the cell in the accommodation unit, according to an evaluation result of the growth stage of the cell of the evaluation unit; and
an image capturing controller unit that controls the image capturing unit such that the image capturing unit captures an image of the cell rotated by the rotation mechanism.

14. The observation system according to claim 13, wherein
the rotation mechanism is a vibration apparatus that vibrates the culture vessel.

15. The observation system according to claim 13, wherein
the accommodation unit is capable of accommodating the cell and a liquid,
the culture vessel includes the rotation mechanism, and
the rotation mechanism rotates the cell by generating a flow in the liquid in the accommodation unit.

16. An information processing method, comprising:
evaluating a growth stage of a cell; and
capturing an image of the rotated cell when it is evaluated that the cell is in a growth stage in which the cell has an asymmetric shape.

17. A program causing an information processing apparatus to execute:
a step of evaluating a growth stage of a cell; and
a step of capturing an image of the rotated cell when it is evaluated that the cell is in a growth stage in which the cell has an asymmetric shape.
